# EUROPEAN PATENT APPLICATION

(11) **EP 4 512 358 A1**
(43) Date of publication of application: **26.02.2025**
(21) Application number: 24192990.0
(22) Date of filing: 06.08.2024
(51) Int. Cl.: A61B 34/37, A61B 17/29

(54) **SURGICAL INSTRUMENT**

(30) Priority: 08.08.2023 JP 2023129606; 08.08.2023 JP 2023146535
(71) Applicant: Medicaroid Corporation, Kobe-shi, Hyogo 650-0047 (JP)
(72) Inventor: TAKAHASHI, Kaoru, Chuo-ku, Kobe-shi, 650-0047 (JP); ITO, Tetsushi, Chuo-ku, Kobe-shi, 650-0047 (JP)
(74) Representative: Müller Hoffmann & Partner

(57) **Abstract**

A surgical instrument 40a for a robotic surgical system 100 according an embodiment includes an end effector 43 including first and second jaws 431 and 432 movable between an open position and a closed position, a wrist joint 45 connected to a proximal end of the end effector 43, and a shaft 42 connected to a proximal end of the wrist joint 45. At least one of the first and second jaw members is rotatably provided about a rotation axis 431a. A diameter D1 of a portion of the end effector 43 where the rotation axis is provided is smaller than a diameter D2 of a central portion in a longitudinal direction of the wrist joint 45 and a diameter D3 of a central portion in a longitudinal direction of the shaft 42.

## Description

### BACKGROUND

The disclosure may relate to a surgical instrument.

In a related art, there has been known a surgical instrument for use in a robotic surgical system. For example, Patent Document 1 discloses a surgical instrument for robotic laparoscopic surgery. The surgical instrument is attached to a robot arm and includes an end effector, a joint portion connected to the end effector, and a shaft connected to the joint portion. Patent Document 1 discloses the shaft has a diameter of 5 mm or less.

Patent Document 1: U.S. Patent Application Publication No. 2019/0223960

### SUMMARY

Although not explicitly stated in Patent Document 1, a surgical instrument with a diameter of 5 mm is the norm in laparoscopic surgery that does not use robots, and there may be a demand for a surgical instrument with a diameter of 5 mm in robotic laparoscopic surgery as well. However, compared to the surgical instrument for the laparoscopic surgery that is manually operated by a doctor, in the surgical instrument for the robotic laparoscopic surgery, a large force may be applied between a trocar (a component for introducing the surgical instrument into a patient's body) and the shaft of the surgical instrument, so the shaft may be required to have great strength. Furthermore, the surgical instrument used for the robotic laparoscopic surgery that includes a wrist joint portion has a more complex structure than the surgical instrument used for the laparoscopic surgery that does not include a wrist joint portion, and thus may have difficultly to reduce the diameter while maintaining the strength thereof.

An object of an embodiment of the disclosure may be to provide a surgical instrument that allows a diameter of an end effector to be reduced while maintaining a strength of the surgical instrument.

An aspect of the disclosure may be a surgical instrument for a robotic surgical system that may include: an end effector including first and second jaw members movable between an open position and a closed position, wherein at least one of the first and second jaw members is rotatably provided about a rotation axis; a wrist joint portion connected to a proximal end of the end effector; and a shaft connected to a proximal end of the wrist joint portion, wherein a diameter D1 of the end effector at a portion where the rotation axis is provided is smaller than a diameter D2 of a central portion in a longitudinal direction of the wrist joint portion and a diameter D3 of a central portion in the longitudinal direction of the shaft.

According to the aspect described above, the diameter D1 of the end effector at the portion where the rotation axis of the first and second jaw members is provided is smaller than the diameter D2 of the central portion in the longitudinal direction of the wrist joint portion and the diameter D3 of the central portion in the longitudinal direction of the shaft. This allows the diameter D1 of the end effector on the distal side of the wrist joint portion to be thinned while maintaining the diameter D2 of the longitudinal central portion of the wrist joint portion and the diameter D3 of the longitudinal central portion of the shaft at diameters that can maintain the strength of the surgical instrument. Therefore, unlike a case where the end effector, the wrist joint portion and the shaft are all made to have the same thin diameter, the end effector can be thinned while maintaining the strength of the surgical instrument. Further, even in a case of the surgical instrument for the robotic surgical system including the wrist joint portion, which has a more complex structure than a surgical instrument for laparoscopic surgery that does not include a wrist joint portion, the end effector can be made thinner while maintaining the strength of the surgical instrument.

According to the disclosure, a diameter of an end effector can be reduced while maintaining a strength of a surgical instrument.

### BRIEF DESCRIPTION OF DRAWINGS

FIG.1 is a diagram illustrating a configuration of a robotic surgical system according to an embodiment;
FIG.2 is a block diagram illustrating a control-related configuration of the robotic surgical system according to an embodiment;
FIG.3 is a diagram illustrating a perspective view of a state where a surgical instrument according to an embodiment is attached to a robot arm through an adaptor;
FIG.4 is a diagram illustrating an exploded perspective view of a state where the surgical instrument according to an embodiment is attached to the robot arm through the adaptor;
FIG.5 is a diagram illustrating a perspective view of the surgical instrument according to an embodiment as seen from below.
FIG.6 is a diagram illustrating a plan view of the surgical instrument according to an embodiment;
FIG.7 is a diagram illustrating a perspective view of a state where a lid part of the surgical instrument according to an embodiment is detached;
FIG.8 is a diagram illustrating a plan view of the state where the lid part of the surgical instrument according to an embodiment is detached;
FIG.9 is a diagram illustrating a cross-sectional view of the state where the lid part of the surgical instrument according to an embodiment is detached;
FIG.10 is a diagram illustrating a side view of an end effector, a wrist joint, and a shaft according to an embodiment;
FIG.11 is a diagram illustrating a cross-sectional view of the end effector, the wrist joint, and the shaft according to an embodiment;
FIG.12 is a diagram illustrating a cross-sectional view for explaining a connection between the end effector and the wrist joint according to an embodiment;
FIG.13 is a diagram illustrating a cross-sectional view of a modified example of the end effector according to an embodiment;
FIG.14 is a diagram illustrating a side view of the end effector in a closed position according to an embodiment;
FIG.15 is a diagram illustrating a cross-sectional view of a third joint component taken along the arrow C1-C1 in FIG. 10;
FIG.16 is a diagram illustrating an exploded perspective view of the end effector and a rod according to an embodiment;
FIG.17 is a diagram illustrating a side view of the rod according to an embodiment;
FIG.18 is a diagram illustrating a sectional view of the rod according to an embodiment;
FIG.19 is a diagram illustrating a perspective view of a guide portion in the wrist joint of the surgical instrument according to an embodiment;
FIG.20 is a diagram illustrating a cross sectional view of the guide portion in the wrist joint of the surgical instrument according to an embodiment;
FIG.21 is a diagram illustrating a perspective view of a memory board and a fixing portion according to an embodiment;
FIG.22 is a diagram illustrating a cross sectional view of the memory board and the fixing portion according to an embodiment;
FIG.23 is a diagram illustrating a perspective view of the wrist joint of the surgical instrument according to an embodiment;
FIG.24 is a diagram illustrating an exploded perspective view of the wrist joint of the surgical instrument according to an embodiment;
FIG.25 is a diagram illustrating a cross sectional view of first and fourth meshing portions of the wrist joint of the surgical instrument according to an embodiment;
FIG.26 is a diagram illustrating a cross sectional view illustrating second and third meshing portions of the wrist joint of the surgical instrument according to an embodiment;
FIG.27 is a diagram illustrating an enlarged view of the first and fourth meshing portions of the wrist joint of the surgical instrument according to an embodiment;
FIG.28 is a diagram illustrating an enlarged view of the second and third meshing portions of the wrist joint of the surgical instrument according to an embodiment;
FIG.29 is a diagram illustrating a view of the wrist joint of the surgical instrument in an unrotated state according to an embodiment;
FIG.30 is a diagram illustrating a view of the wrist joint of the surgical instrument in a state where the wrist joint is rotated by an angle θ1 according to an embodiment;
FIG.31 is a diagram illustrating a view of the wrist joint of the surgical instrument in a state where the wrist joint is rotated by an angle θ2 according to an embodiment; and
FIG.32 is a diagram illustrating a view of the wrist joint of the surgical instrument in a state where the wrist joint is rotated by an angle θ3 according to an embodiment.

### DETAILED DESCRIPTION

Descriptions are provided hereinbelow for embodiments based on the drawings. In the respective drawings referenced herein, the same constituents are designated by the same reference numerals and duplicate explanation concerning the same constituents is omitted. All of the drawings are provided to illustrate the respective examples only.

### (Configuration of Robotic Surgical System)

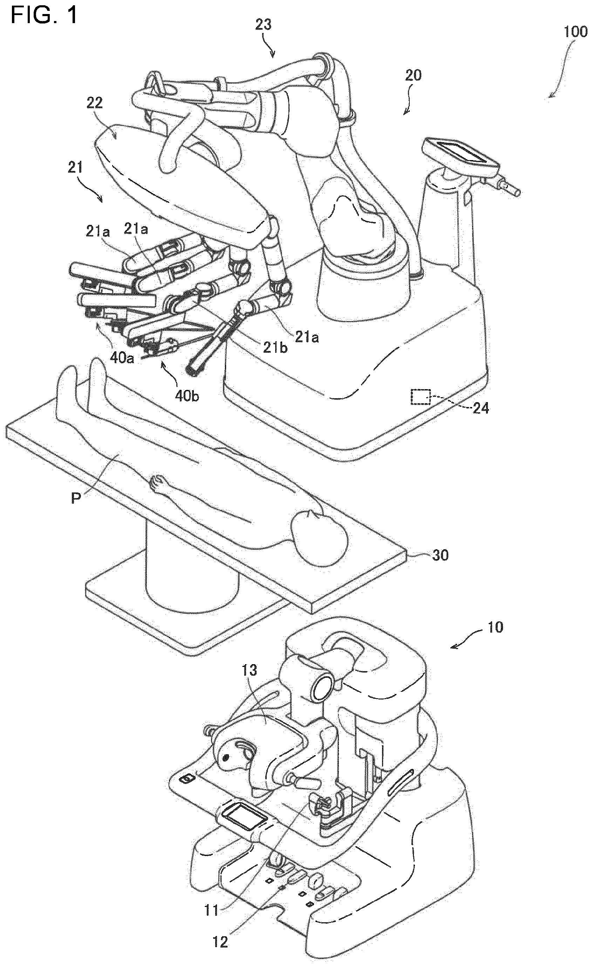

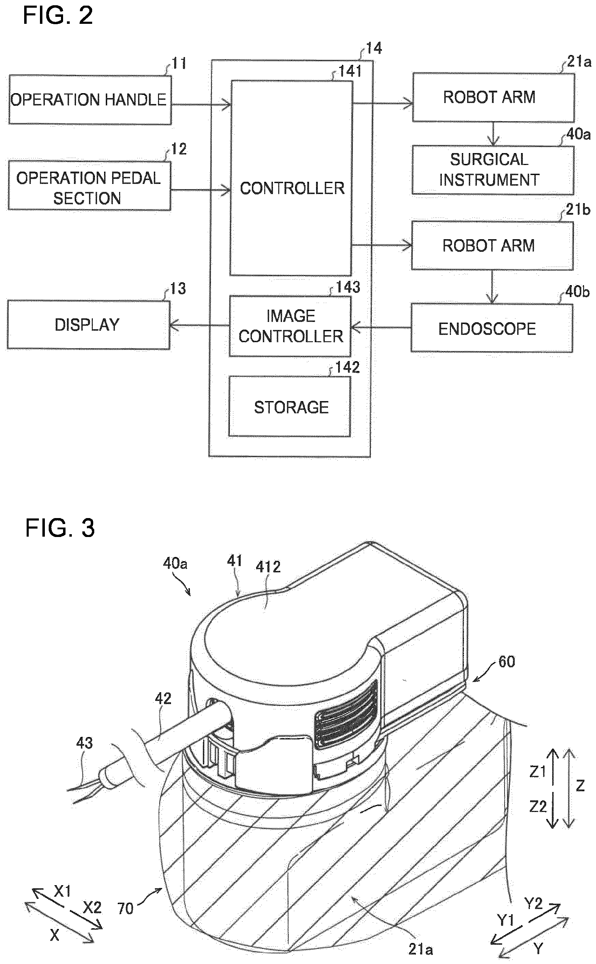

A configuration of a robotic surgical system 100 according to an embodiment is described with reference to FIGS. 1 and 2.

As illustrated in FIG. 1, the robotic surgical system 100 includes a remote control apparatus 10 and a patient-side apparatus 20. The remote control apparatus 10 is provided to remotely control medical equipment attached to the patient-side apparatus 20. When an operator, as a surgeon, inputs an action mode instruction to be executed by the patient-side apparatus 20, to the remote control apparatus 10, the remote control apparatus 10 transmits the action mode instruction to the patient-side apparatus 20 through a controller 24. In response to the action mode instruction transmitted from the remote control apparatus 10, the patient-side apparatus 20 operates the medical equipment, including surgical instruments 40a attached to robot arms 21a and an endoscope 40b attached to a robot arm 21b.

The patient-side apparatus 20 is positioned beside an operation table 30 on which the patient P is laid. The patient-side apparatus 20 includes plural robot arms 21a and 21b. One (21b) of the robot arms holds the endoscope 40b and the other robot arms (21a) hold the surgical instruments 40a. Each of the plural robot arms 21a and 21b includes plural joints. Each joint includes a driver (a driving device) including a servo-motor and a position detector such as an encoder or the like.

The arm base 22 is supported by a positioner 23 placed on the floor of an operation room. The positioner 23 includes a vertical articulated robot. The positioner 23 is configured to move the position of the arm base 22 three-dimensionally. The controller 24 is a control circuit including an arithmetic unit such as a CPU and/or the like, and a memory such as a ROM, a RAM, and/or the like.

The surgical instruments 40a as the medical equipment are detachably attached to the distal ends of the robot arms 21a. The surgical instrument 40a includes a housing 41 (see FIG. 3), an elongate shaft 42 (see FIG. 3), and an end effector 43 (see FIG. 3). The end effector 43 may be, for example, forceps, but is not limited to this. The end effector 43 may be any one or more of various treatment tools, or the like. In surgeries using the patient-side apparatus 20, the robot arms 21a introduce the surgical instruments 40a into the body of the patient P through a cannula (trocar) placed on the body surface of the patient P.

To the distal end of the robot arm 21b, the endoscope 40b as the medical equipment is detachably attached. The endoscope 40b captures an image in a body cavity of the patient P. The captured image is outputted to the remote control apparatus 10. The endoscope 40b may be a 3D endoscope capable of capturing a three-dimensional image or a 2D endoscope. In surgeries using the patient-side apparatus 20, the robot arm 21b introduces the endoscope 40b into the body of the patient P through a trocar placed on the body surface of the patient P.

The remote control apparatus 10 is an apparatus that allows the operator to operate the medical equipment attached to the robot arms 21a and 21b. Specifically, the remote control apparatus 10 is configured to transmit action mode instructions which are inputted by the operator and are to be executed by the surgical instruments 40a and the endoscope 40b, to the patient-side apparatus 20 through the controller 24.

The action modes to be executed by the surgical instruments 40a include modes of actions to be taken by each surgical instrument 40a (a series of positions and postures) and actions to be executed by the function of each surgical instrument 40a. For example, in a case in which the surgical instrument 40a is a vessel sealer, the operational modes to be performed by the surgical instrument 40a include pitch and yaw rotation of the wrist of the end effector 43, opening and closing of the jaws, coagulating tissue by supplying a coagulation current to the end effector 43, and incising tissue by supplying a cutting current to the end effector 43.

The action modes to be executed by the endoscope 40b include the position and posture of the distal end of the endoscope 40b and setting of the zoom magnification, for example.

As illustrated in FIGS. 1 and 2, the remote control apparatus 10 includes operation handles 11, an operation pedal section 12, a display 13, and a control apparatus 14.

The operation handles 11 are provided in order to remotely operate medical equipment attached to the robot arms 21a. Specifically, the operation handles 11 accept operations by the operator for operating the medical equipment (the surgical instruments 40a and endoscope 40b). The operation handles 11 are composed of two operation handles 11 arranged side by side in the horizontal direction.

The operation handles 11 extend from the rear side of the remote control apparatus 10 toward the front side. The operation handles 11 are configured to move in a predetermined three-dimensional operation region.

The remote control apparatus 10 and the patient-side apparatus 20 constitute a master-slave system in terms of controlling movement of the robot arms 21a and 21b. The operation handles 11 constitute a master side operating part in the master-slave system, and the robot arms 21a and 21b to which the medical equipment are attached constitute a slave side operating part in the master-slave system. When the operator operates the operation handles 11, the movement of one of the robot arms 21a or 21b is controlled so that the distal end portion (the end effector 43 of the surgical instrument 40a) of the robot arm 21a or the distal end portion (the endoscope 40b) of the robot arm 21b moves following the movement of the operation handles 11.

The operation pedal section 12 or an operation pedal unit includes plural pedals to execute medical equipment-related functions. The plural pedals include a coagulation pedal, a cutting pedal, a camera pedal, and a clutch pedal. The plural pedals are operated by a foot of the operator.

By operating the coagulation pedal, a coagulation current is supplied to the surgical instrument 40a so as to coagulate the surgical site. By operating the cutting pedal, a cutting current is supplied to the surgical instrument 40a so as to cut the surgery site.

The camera pedal, when operated, enables the operation handles 11 to operate the endoscope 40b. That is, the position and orientation of the endoscope 40b are controllable by the operation handles 11 while the camera pedal is being pressed. The endoscope 40b is controlled by using both of the right and left operation handles 11, for example.

The clutch pedal is used to temporarily disconnect operation-related connection between the operation handles 11 and the robot arms 21a to stop movement of the surgical instruments 40a. Specifically, when the clutch pedal is being pressed, the robot arms 21a of the patient-side apparatus 20 do not work even if the operation handles 11 are operated.

The display 13 (or a display device) is configured to display images captured by the endoscope 40b. The display 13 comprises a scope type display or a non-scope type display. (Note that FIG. 1 illustrates a scope type display 13.) The scope type display is a display configured in such a manner that the operator looks into the display. The non-scope type display is a display like an open-type display that includes a flat screen and the operator is able to see without looking into, such as normal displays for personal computers.

The display 13 displays a 3D image captured by the endoscope 40b attached to the robot arm 21b of the patient-side apparatus 20. Note that, the display 13 may display a 2D image captured by the endoscope 40b provided to the patient-side apparatus 20.

As illustrated in FIG. 2, the control apparatus 14 includes a controller 141, a storage 142, and an image controller 143, for example. The controller 141 includes an arithmetic unit such as a CPU. The storage 142 includes a memory, such as a ROM and a RAM. The control apparatus 14 may be composed of a single control apparatus performing centralized control or may be composed of plural control apparatuses that perform decentralized control in cooperation with each other. The controller 141 determines whether an action mode instruction inputted by the operation handles 11 is to be executed by the robot arms 21a or to be executed by the endoscope 40b, depending on the state of the operation pedal section 12. When determining that the action mode instruction inputted by the operation handles 11 is to be executed by any one of the surgical instruments 40a, the controller 141 transmits the action mode instruction to the corresponding robot arm 21a through the controller 24. The robot arm 21a is thereby driven by the controller 24 and thus the movement of the surgical instrument 40a attached to the robot arm 21a is controlled.

The storage 142 stores control programs corresponding to the types of the surgical instrument 40a, for example. The controller 141 reads the stored control programs according to the types of the attached surgical instruments 40a. The action mode instructions from the operation handles 11 and/or the operation pedal section 12 of the remote control apparatus 10 thereby cause the respective surgical instruments 40a to perform proper movements.

The image controller 143 transmits images acquired by the endoscope 40b to the display 13. The image controller 143 performs processing and modifying the images when needed.

### (Configurations of Surgical Instrument, Adaptor, Drape, and Robot Arm)

With reference to FIGS. 3 to 5, configurations of the surgical instrument 40a, an adaptor 60, a drape 70, and the robot arm 21a are described.

Here, a longitudinal direction of the surgical instrument 40a (a longitudinal direction of the shaft 42) is defined as the Y direction, the distal side (the side toward the end effector 43) of the surgical instrument 40a along the Y direction is defined as the Y1 direction, and the opposite side of the Y1 direction is defined as the Y2 direction. The direction in which the surgical instrument 40a and the adaptor 60 are adjacent to each other is defined as a Z direction, the surgical instrument 40a side along the Z direction is defined as a Z1 direction, and the opposite side of the Z1 direction is defined as a Z2 direction. Further, the direction orthogonal to the Y direction and the Z direction is referred to as an X direction, one side along the X direction is referred as an X1 direction, and the other side along the X direction is referred to as an X2 direction.

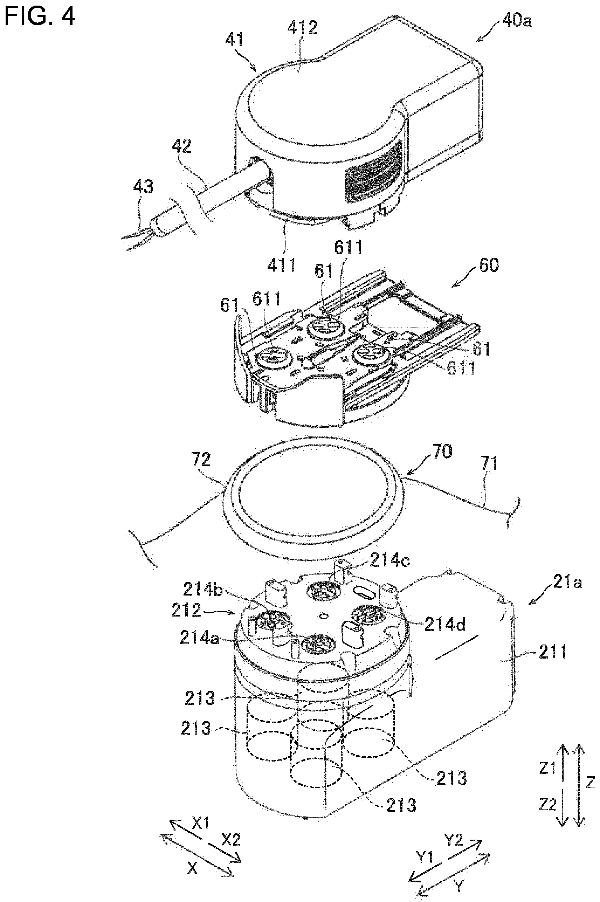

As illustrated in FIGS. 3 and 4, the surgical instrument 40a is detachably attached to the robot arm 21a of the robotic surgical system 100. Specifically, the surgical instrument 40a is detachably attached to the robot arm 21a via the adaptor 60. The adaptor 60 is a drape adaptor configured to sandwich a sterile drape 70 to cover the robot arm 21a, in conjunction with the robot arm 21a.

The surgical instrument 40a is attached to the Z1 side of the adaptor 60. The adaptor 60 is attached to the Z1 side of the robot arm 21a.

As illustrated in FIG. 4, the drape 70 includes a body section 71 that covers the robot arm 21a and an attachment section 72 sandwiched between the robot arm 21a and the adaptor 60. The body section 71 is made of a flexible film member. The body section 71 includes an opening so that the robot arm 21a is engaged with the adaptor 60. To the opening of the body section 71, the attachment section 72 is provided. The attachment section 72 is made of a resin mold member. The attachment section 72 is harder (less flexible) than the body section 71. The attachment section 72 includes an opening so that the robot arm 21a is engaged with the adaptor 60.

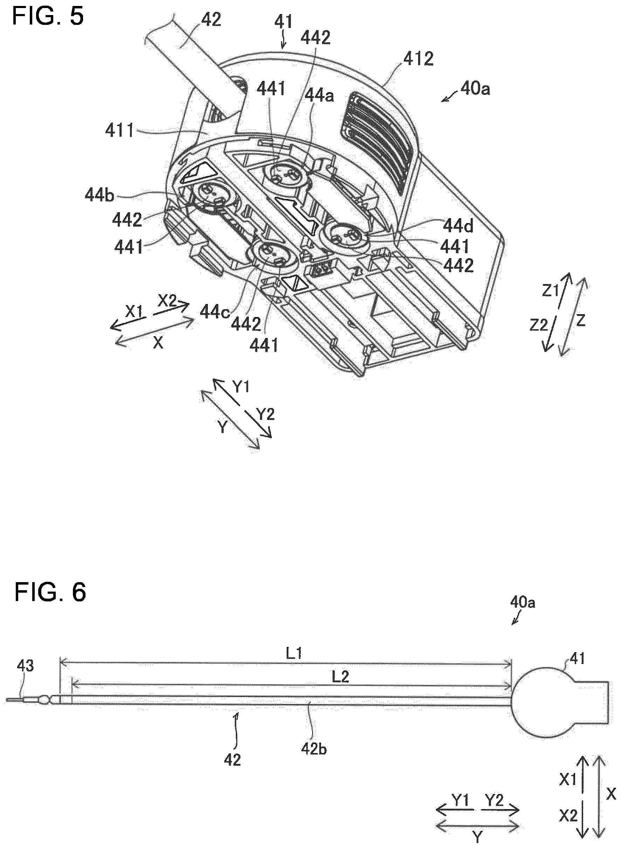

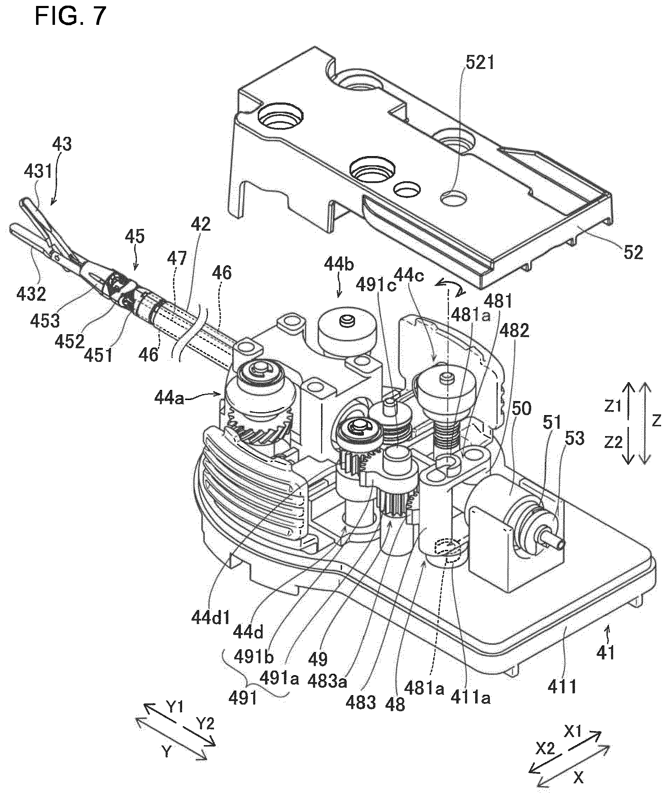

As illustrated in FIGS. 5 and 7, the surgical instrument 40a includes a plurality (four) of drive shafts 44a, 44b, 44c and 44d. The drive shafts 44a, 44b, 44c, and 44d are provided within the housing 41 and are rotatable about respective rotation axes extending along the Z axis. The drive shafts 44a to 44d are rotationally driven by driving forces from motors of drivers 213 (driving devices 213) provided in the robot arm 21a. The drive shafts 44a to 44d are provided for operating (driving) the elongate shaft 42, the end effector 43, and a wrist joint 45 (described later).

The drive shafts 44a to 44d respectively include reception members 442 engaged with corresponding drive transmission members 61 (described later) of the adaptor 60 so as to transmit the driving forces from the robot arm 21a to the shaft 42, the end effector 43, and the wrist joint 45. Each of the reception members 442 is provided with a protrusion 441 that is engaged with a recess formed in the corresponding drive transmission member 61, and the protrusion 441 protrudes from the Z2 side surface of each of the drive shafts 44a to 44d toward the adaptor 60 (the Z2 side). Each of the protrusions 441 includes plural protrusion portions that arranged in a straight line. The reception member 442 of the drive shaft 44d is an example of a "first reception member." Moreover, the reception members 442 of the drive shafts 44b and 44c are examples of "second reception members."

As illustrated in FIG. 4, the adaptor 60 includes the drive transmission members 61. The drive transmission members 61 are configured to transmit the driving forces from the robot arm 21a to the drive shafts 44a to 44d of the surgical instrument 40a. In other words, the drive transmission members 61 respectively provided to correspond to the drive shafts 44a to 44d of the surgical instrument 40a. The drive transmission members 61 are rotatable about the respective rotation axes, which extend along the Z direction.

The drive transmission members 61 include engagement recesses 611 that are engaged with the protrusions 441 of the respective drive shafts 44a to 44d of the surgical instrument 40a. The engagement recess 611 is located at the surgical instrument 40a side (the Z1 side) of the drive transmission member 61 and is recessed from the Z1 side surface of the drive transmission member 61, toward the Z2 direction, opposite to the surgical instrument 40a. The drive transmission members 61 each include, on a Z2 side surface thereof, an engagement recess that is engaged with an engagement protrusion of a corresponding one of drive members 214a to 214d (described later) of the robot arm 21a.

The robot arm 21 a includes a frame 211 and an instrument attachment portion 212 (or an instrument mount 212). The instrument attachment portion 212 includes drivers (driving devices) 213 and drive members 214a to 214d. The plural (four) drivers 213 are provided corresponding to the plural (four) drive shafts 44a to 44d of the surgical instrument 40a and corresponding to the plural (four) drive transmission members 61 of the adaptor 60. Each of the drivers 213 includes an absolute encoder and a servomotor and is configured to drive the corresponding driving member to rotate about a rotational axis thereof extending in the Z direction. The plural drive members 214a to 214d correspond to the plural drive shafts 44a to 44d, respectively, and each drive member includes the engagement protrusion that is engaged with the engagement recess on the Z2 side surface of the corresponding drive transmission member 61. The drivers 213 are configured to drive the drive transmission members 61 of the adaptor 60, which are respectively engaged with the drive members 214a to 214d of the drivers 213, to rotate about the rotational axes of the drive transmission members 61 extending in the Z direction, so as to drive the drive shafts 44a to 44d of the surgical instrument 40a, which are respectively engaged with the drive transmission members 61, to rotate about the rotational axes of the drive shafts 44a to 44d extending in the Z direction. The drive member 214d is an example of a "first drive member." The drive members 214b and 214c are examples of "second drive members."

The drive shaft 44d receives a driving force for driving the end effector 43 from the drive member 214d. The drive shafts 44b and 44c receive driving forces for driving the wrist joint 45 from the drive members 214b and 214c. The drive shaft 44a receives a driving force for driving the shaft 42 from the drive member 214a.

### (Detailed Configuration of Surgical Instrument)

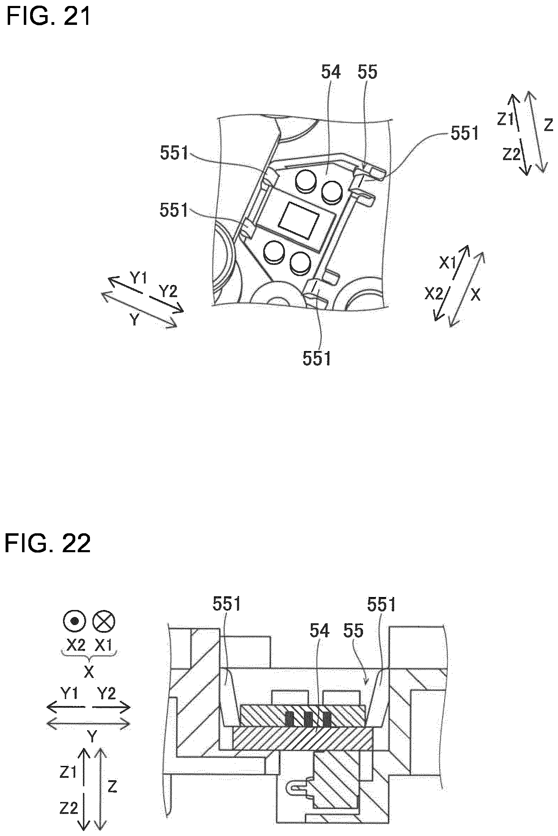

With reference to FIGS. 6 to 32, the configuration of the surgical instrument 40a is described in detail below.

As illustrated in FIGS. 6 to 32, the surgical instrument 40a includes the housing 41, the elongate shaft 42, the end effector 43, and the plural drive shafts 44a to 44d. The surgical instrument 40a also includes the wrist joint 45, wires 46, a rod 47, a lever 48, a driving force transmission part 49, a holding member 50, and a spring member 51. The housing 41 is an example of an "interface." The wrist joint 45 is an example of a "wrist joint portion." The rod 47 is an example of an "elongate element".

As illustrated in FIG. 6, the housing 41 is disposed on a proximal end side (Y2 side) of the shaft 42. In addition, the housing 41 is attached to the instrument attachment portion 212 of the robot arm 21a. As illustrated in FIG. 7, the housing 41 includes a base 411 and a lid part 412 (see FIG. 4). The base 411 is attached to the robot arm 21a (see FIG. 4) via the adaptor 60. The base 411 is provided with the plural drive shafts 44a to 44d. The lid part 412 removably covers the base 411. Specifically, the lid part 412 covers the base 411 from the side (the Z1 side) opposite to a mounting surface of the base 411 to the robot arm 21a.

The shaft 42 is provided to extend in the Y direction and has a hollow cylindrical shape. A proximal end (an end on the Y2 side) of the shaft 42 is connected to the base 411. A distal end (an end on the Y1 side) of the shaft 42 is connected to a proximal end of the wrist joint 45.

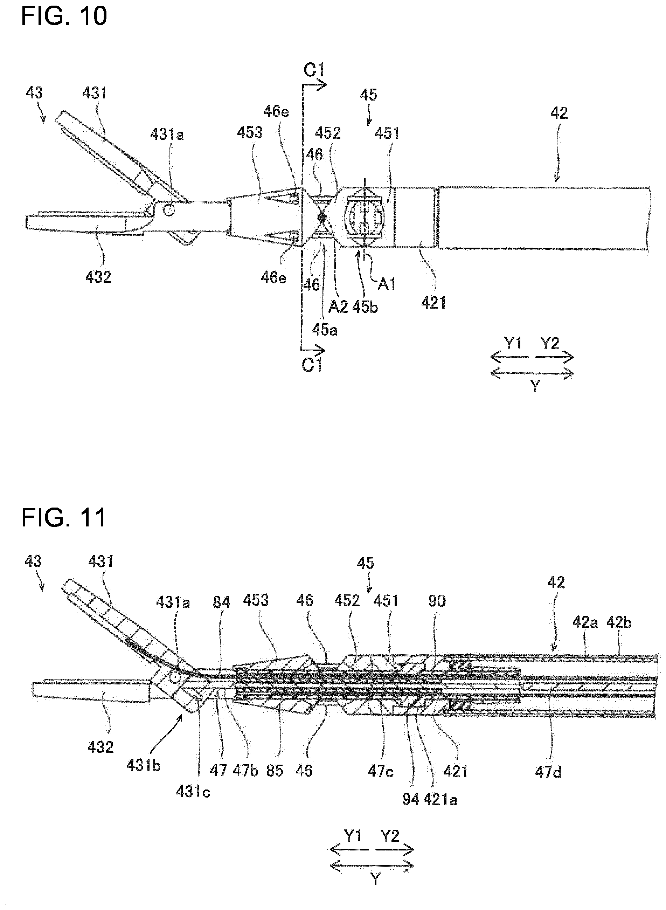

As illustrated in FIG. 11, the shaft 42 includes a hollow metal shaft 42a and a heat shrink tube 42b (a heat shrinkable tube) that covers the surface of the metal shaft 42a. The heat shrink tube 42b is heated to be shrunk to cover the surface of the metal shaft 42a.

The heat shrink tube 42b covers 90% or more and 100% or less of the metal shaft 42a in the longitudinal direction (Y direction) of the metal shaft 42a. This makes it possible to effectively prevent the material (the heat shrink tube 42b) covering the shaft 42 from peeling off when covering a wide area of the metal shaft 42a with the heat shrink tube 42b. It may be preferable that the heat shrink tube 42b covers 95% or more and 99% or less of the metal shaft 42a in the longitudinal direction of the metal shaft 42a.

For example, as illustrated in FIG. 6, the metal shaft 42a has a length L1 in the Y direction. The heat shrink tube 42b has a length L2 in the Y direction. The following equation is satisfied: 0.90*L1 & L2 & L1. It may be preferable that the following equation is satisfied: 0.95*L1 & L2 & 0.99*L1. In a case where there is a portion of the metal shaft 42a that is not covered with the heat shrink tube 42b, the portion of the metal shaft 42a that is not covered with the heat shrink tube 42b is provided on a distal end side of the metal shaft 42a.

The heat shrink tube 42b has insulating properties. This allows the surface of the metal shaft 42a to be covered with an insulating coating (the heat shrink tube 42b). This effect is particularly effective, when the surgical instrument 40a is an electrosurgical instrument as in an embodiment, since the metal shaft 42a may be required to have insulating properties.

The metal shaft 42a is a stainless steel pipe. The heat shrink tube 42b is a polyethylene-based heat shrink tube or a fluororesin-based heat shrink tube. As a result, since the metal shaft 42a is a high-strength stainless steel pipe, the shaft 42 can be ensured to have high strength. Further, since the heat-shrink tube 42b is a low-friction polyethylene-based heat-shrink tube or fluororesin-based heat-shrink tube, peeling of the material (the heat-shrink tube 42b) covering the shaft 42 due to friction between the cannula (trocar) and the shaft 42 can be prevented. This effect is particularly effective in a case of a surgical robot in which the robot arm 21a does not have a mechanism for gripping a cannula (trocar) as in an embodiment.

The heat shrink tube 42b has a single layer structure. This allows the structure of the heat shrink tube 42b to be simpler than a case in which the heat shrink tube 42b has a two-layer structure.

The heat shrink tube 42b is black. This makes it possible to suppress illumination light from the endoscope 40b from reflecting on the shaft 42, thereby making it possible to suppress occurrence of halation.

As illustrated in FIG. 7, the end effector 43 is connected to a distal end of the wrist joint 45. The end effector 43 includes a first jaw 431 and a second jaw 432 that are rotatably provided about a rotation axis (a support shaft 431a, described later) and thus are rotatable about the rotation axis so as to move relative to each other between an open position and a closed position. The first jaw 431 and the second jaw 432 each include a conductor. For example, the first jaw 431 and the second jaw 432 are made of stainless steel, which is a conductor. Further, the first jaw 431 and the second jaw 432 are insulated from each other so as not to be electrically connected to each other. The first jaw 431 and the second jaw 432 are examples of a "first jaw member" and a "second jaw member", respectively.

An outer surface of a portion of the first jaw 431 that is not opposed to the second jaw 432 is covered with an electrically insulating material. An outer surface of a portion of the second jaw 432 that is not opposed to the first jaw 431 is covered with an electrically insulating material. An outer surface of the wrist joint 45 is coated with an electrically insulating material. With this configuration, the outer surfaces of the non-opposing portions of the first jaw 431 and the second jaw 432 are prevented from being exposed to the outside, and thus discharge through the outer surfaces of the non-opposing portions of the first jaw 431 and the second jaw 432 can be prevented. Further, the outer surface of the wrist joint 45 is prevented from being exposed to the outside, and thus discharge through the outer surface of wrist joint 45 can be prevented.

As illustrated in FIGS. 7 to 9, the drive shafts 44a to 44d include a first drive shaft 44a that rolls the shaft 42 about an axis thereof in the Y direction, a second drive shaft 44b and a third drive shaft 44c that pitch the wrist joint 45 about a first direction (X direction in FIG. 7) orthogonal to the Y direction and yaw the wrist joint 45 about a second direction (Z direction in FIG. 7) orthogonal to the Y direction and the first direction, and a fourth drive shaft 44d that moves the rod 47 in the Y direction.

The first drive shaft 44a is connected to a gear 422 that is connected to the proximal end of the shaft 42. When the first drive shaft 44a is driven to rotate, the elongate shaft 42 is driven to rotate in a rolling manner by the first drive shaft 44a via the gear 422. The second drive shaft 44b and the third drive shaft 44c are connected to the wires 46 connected to the wrist joint 45. When the second drive shaft 44b and the third drive shaft 44c are driven to rotate, the wrist joint 45 is caused to perform pitch or yaw joint motion via the wires 46. Note that the movement of the rod 47 in the Y direction by the fourth drive shaft 44d will be described later.

As illustrated in FIGS. 7, 10 and 11, the wrist joint 45 is connected to the proximal end of the end effector 43 and is also connected to the distal end side (Y1 side) of the shaft 42 so as to be capable of articulation. The wrist joint 45 also includes a conductor. For example, the wrist joint 45 is made of stainless steel, which is a conductor. Specifically, the wrist joint 45 includes a first joint component 451, a second joint component 452, and a third joint component 453. The first joint component 451 (or a first joint part 451) is connected to the distal end side (Y1 side) of the shaft 42. The second joint component 452 (or a second joint part 452) is disposed on a distal end side (Y1 side) of the first joint component 451, and is meshed with the first joint component 451 so as to perform a yaw joint movement. The third joint component 453 (or a third joint part 453) is disposed on a distal end side of the second joint component 452 and is meshed with the second joint component 452 so as to perform a pitch joint movement. The meshing in the wrist joint 45 will be described in detail later. The first joint component 451 is an example of a "third member." The second joint component 452 is an example of a "second member." The third joint component 453 is an example of a "first member."

The wrist joint 45 is configured to articulate with multiple degrees of freedom. This allows the end effector to move more freely since the wrist joint 45 can articulate with the multiple degrees of freedom. Specifically, the wrist joint 45 includes a first joint 45a between the third joint component 453 including a connection portion 45c (described later) including a conductor and the second joint component 452, and the wrist joint 45 also includes a second joint 45b between the second joint component 452 and the first joint component 451. The first joint 45a is configured to articulate about a rotation axis A2 that intersects with the longitudinal direction (Y direction) of the shaft 42. The first joint 45a is configured to articulate with a degree of freedom in a pitch direction. The second joint 45b is configured to articulate about a rotation axis A1 that intersects both the longitudinal direction (Y direction) of the shaft 42 and the rotation axis A2. The second joint 45b is configured to articulate with a degree of freedom in a yaw direction. In addition, the degree of freedom in the pitch direction is the degree of freedom in a direction orthogonal to the longitudinal axis (Y-axis) of the shaft 42, and the degree of freedom in the yaw direction is the degree of freedom in a direction orthogonal to the longitudinal axis and the pitch direction. The rotation axes A1 and A2 are examples of a "second rotation axis" and a "first rotation axis", respectively.

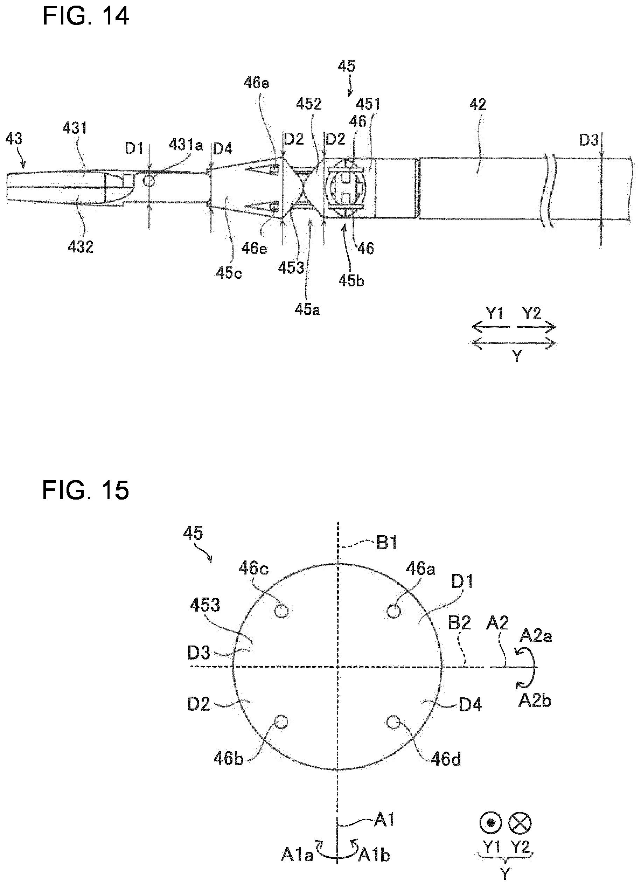

As illustrated in FIG. 14, a diameter D1 of the end effector 43 at a portion where the rotation axis (the support shaft 431a described later) of the first jaw 431 and the second jaw 432 is provided is smaller than a diameter D2 of a central portion in the longitudinal direction (Y direction) of the wrist joint 45 and a diameter D3 of a central portion in the longitudinal direction (Y direction) of the shaft 42. This allows the diameter D1 of the end effector 43 on the distal side of the wrist joint 45 to be reduced while maintaining the diameter D3 of the central portion of the shaft 42 at a diameter sufficient to maintain strength. Therefore, unlike a case in which the end effector 43, the wrist joint 45 and the shaft 42 are all made to have the same small diameter, the end effector 43 can be made thinner while maintaining the strength of the surgical instrument. Further, even in the case where the surgical instrument 40a for the robotic surgical system includes the wrist joint 45, which has a more complex structure than a surgical instrument for laparoscopic surgery that does not have the wrist joint 45, the end effector 43 can be made thinner while maintaining the strength of the surgical instrument. The diameter D2 is substantially the same as the diameter D3. Further, the diameter D1 is the diameter in the state where the first jaw 431 and the second jaw 432 are closed to each other.

The diameter D1 is greater than 1/2 (4/8) of the diameters D2 and D3 and smaller than 3/4 (6/8) of the diameters D2 and D3. With this configuration, since the diameter D1 is larger than half of the diameters D2 and D3, it is possible to prevent the diameter D1 from becoming excessively small. Further, since the diameter D1 is smaller than 3/4 of the diameters D2 and D3, it is possible to prevent the diameter D1 from becoming excessively large. As a result, the end effector 43 can be made to an appropriate diameter. The diameter D1 is not particularly limited, but is, for example, 5/8 of the diameters D2 and D3.

Further, the diameter D1 is not less than 4 mm and not more than 6 mm. Further, the diameter D2 and the diameter D3 are not less than 7 mm and not more than 9 mm. This allows the end effector 43, the wrist joint 45, and the shaft 42 to be made to appropriate diameters. The diameter D1 is not particularly limited, but is, for example, 5 mm. Further, the diameters D2 and D3 are not particularly limited, but are, for example, 8 mm.

The wrist joint 45 also includes the connection portion 45c for connecting to the end effector 43 and to which the end effector 43 is connected, and a diameter D4 of a distal end of the connection portion 45c is smaller than the diameters D2 and D3. This allows the diameter D4 of the distal end of the connection portion 45c of the wrist joint 45 with the end effector 43 to be matched with the diameter D1 of the end effector 43, making it easy to connect the end effector 43 and the wrist joint 45. The connection portion 45 c is provided to the third joint component 453 of the wrist joint 45.

The connection portion 45c of the wrist joint 45 has the diameter D4 at the distal end of the connection portion 45c and the diameter D2 at a proximal end of the connection portion 45c. This allows the diameter of the connection portion 45c of the wrist joint 45 to be reduced from D2 to D4, making it easy to reduce the diameter of the end effector 43 while maintaining the strength. The connection portion 45c of the wrist joint 45 has a shape that gradually tapers from the proximal end having the diameter D2 to the distal end having the diameter D4. Further, the diameter D4 of the connection portion 45c is substantially the same as the diameter D1.

The wires 46 are provided to drive the wrist joint 45. The wires 46 allow the wrist joint 45 to perform the pitch and yaw joint movements. The wires 46 are provided inside the shaft 42 and extend in the Y direction and the number of the wires 46 provided is four. Two of the four wires 46 connect the wrist joint 45 and the second drive shaft 44b to each other, and the other two of the four wires 46 connect the wrist joint 45 and the third drive shaft 44c to each other.

For example, as illustrated in FIG. 15, the four wires 46 include wires 46a, 46b, 46c, and 46d. The wires 46a and 46b have distal ends thereof connected to the wrist joint 45 and proximal ends thereof connected to the second drive shaft 44b. The wires 46a and 46b are wound around the second drive shaft 44b in opposite directions. In other words, when the second drive shaft 44b rotates in one direction, one of the wires 46a and 46b is pulled in the proximal direction (Y2 direction), and the other of the wires 46a and 46b is released and pulled out in the distal direction (Y1 direction). To the contrary, when the second drive shaft 44b rotates in the other direction opposite to the one direction, the other of the wires 46a and 46b is pulled in the proximal direction (Y2 direction), and the one of the wires 46a and 46b is released and pulled out in the distal direction (Y1 direction).

The wires 46c and 46d have distal ends thereof connected to the wrist joint 45 and proximal ends thereof connected to the third drive shaft 44c. The wires 46c and 46d are wound around the third drive shaft 44c in opposite directions. In other words, when the third drive shaft 44c rotates in one direction, one of the wires 46c and 46d is pulled in the proximal direction (Y2 direction), and the other of the wires 46c and 46d is released and pulled out in the distal direction (Y1 direction). To the contrary, when the third drive shaft 44c rotates in the other direction opposite to the one direction, the other of the wires 46c and 46d is pulled in the proximal direction (Y2 direction), and the one of the wires 46c and 46d is released and pulled out in the distal direction (Y1 direction).

As illustrated in FIG. 10, the wrist joint 45 includes the second joint 45b that rotates about the rotation axis A1, and the first joint 45a that rotates about the rotation axis A2. The wires 46 cause the wrist joint 45 to pitch and yaw, by driving the wires 46a and 46b by the second drive shaft 44b and by driving the wires 46c and 46d by the third drive shaft 44c. The wrist joint 45 is driven by the second drive shaft 44b and the third drive shaft 44c to perform the rotation (the yaw joint movement) about the rotation axis A1 of the second joint 45b and the rotation (the pitch joint movement) about the rotation axis A2 of the first joint 45a. Specifically, as illustrated in FIG. 15, when the wire 46a is pulled and the wire 46b is loosened by the second drive shaft 44b and the wire 46d is pulled and the wire 46c is loosened by the third drive shaft 44c, the second joint 45b rotates in the A1b direction about the rotation axis A1. Further, when the wire 46b is pulled and the wire 46a is loosened by the second drive shaft 44b and the wire 46c is pulled and the wire 46d is loosened by the third drive shaft 44c, the second joint 45b rotates in the A1a direction about the rotation axis A1.

To the contrary, when the wire 46a is pulled and the wire 46b is loosened by the second drive shaft 44b and the wire 46c is pulled and the wire 46d is loosened by the third drive shaft 44c, the first joint 45a rotates in the A2a direction about the rotation axis A2. Further, when the wire 46b is pulled and the wire 46a is loosened by the second drive shaft 44b and the wire 46d is pulled and the wire 46c is loosened by the third drive shaft 44c, the first joint 45a rotates in the A2b direction about the rotation axis A2.

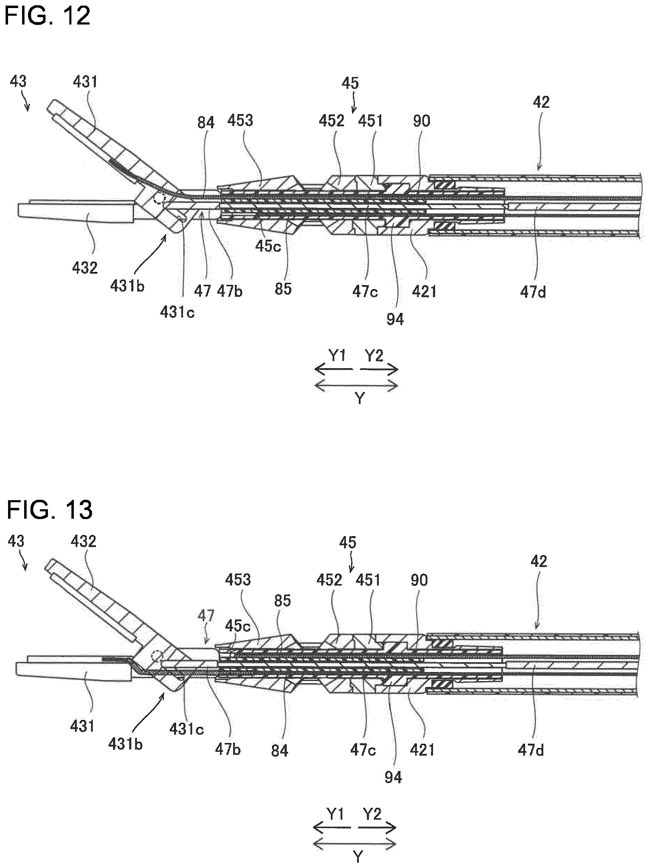

Further, the four wires 46 a to 46d have their ends 46e (see FIG. 10) fixed to the third joint component 453 at the vicinity of the proximal end of the third joint component 453. FIG. 15 is a cross sectional view of the third joint component 453 taken along the line C1-C1 of FIG. 10. As illustrated in FIG. 15, a cross section of the third joint component 453 includes four regions, which are a first region R1 to a fourth region R4, defined by a plane B1 parallel to both the longitudinal direction (Y direction) of the shaft 42 and the rotation axis A1, and a plane B2 parallel to both the longitudinal direction (Y direction) of the shaft 42 and the rotation axis A2. The four wires 46a to 46d pass through the first region D1 to the fourth region D4, respectively, and have the ends 46e thereof fixed to the vicinity of the proximal end of the third joint component 453. Specifically, the wire 46a passes through the first region D1 at a position offset from planes B1 and B2, the wire 46b passes through the second region D2 at a position offset from planes B1 and B2, the wire 46c passes through the third region D3 at a position offset from planes B1 and B2, and the wire 46d passes through the fourth region D4 at a position offset from planes B1 and B2. As illustrated in FIG. 12, the first region D1 is arranged in a first quadrant, the second region D2 is arranged in a third quadrant, the third region D3 is arranged in a second quadrant, and the fourth region D4 is arranged in a fourth quadrant.

As illustrated in FIGS. 7 to 9 and 11, the rod 47 is provided for driving the end effector 43. The rod 47 is provided inside the wrist joint 45 and the shaft 42 so as to extend in the Y direction. A distal end of the rod 47 is connected to the end effector 43. Specifically, the distal end of the rod 47 is connected to the first jaw 431.

The rod 47 is configured to move inside the wrist joint 45 and the shaft 42 in the longitudinal direction (Y direction) of the shaft 42, thereby moving the first jaw 431 and the second jaw 432 relative to each other between the open position and the closed position. This allows the first jaw 431 and the second jaw 432 to be easily moved between the open position and the closed position relative to each other using the wrist joint 45 and the rod 47 that moves inside the shaft 42 in the longitudinal direction of the shaft 42. The rod 47 includes a conductor.

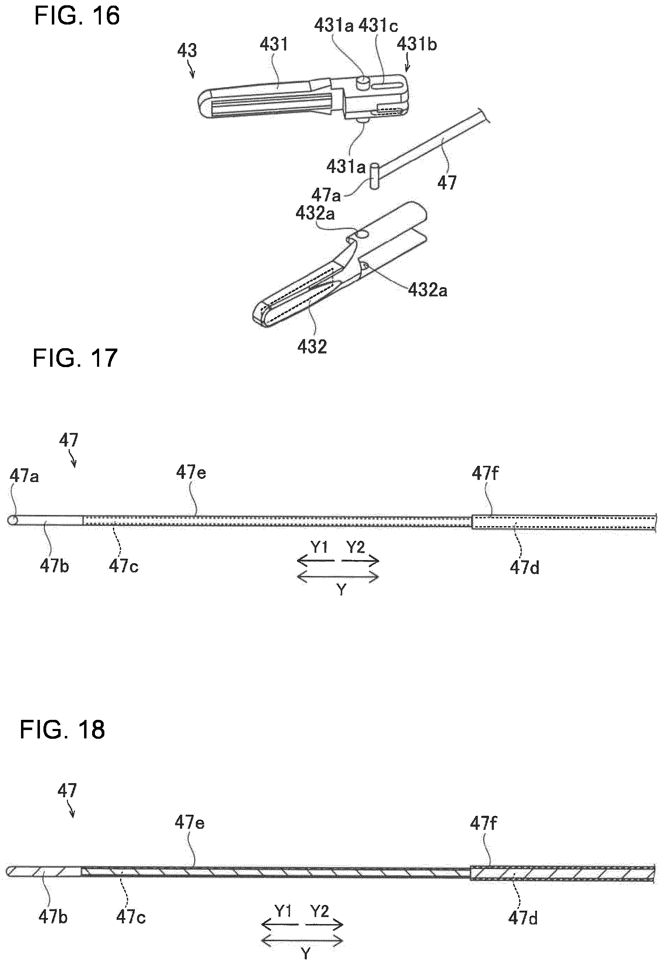

As illustrated in FIGS. 11 and 16, the first jaw 431 includes the support shaft 431a that is rotatably supported by the second jaw 432 and a connection portion 431b that is connected to the distal end of the rod 47, such that the first jaw 431 rotates about an axis of the support shaft 431a in response to the movement of the rod 47 in the Y direction. With this, the first jaw 431 can be rotated about the axis of the support shaft 431a by moving the rod 47 in the Y direction, and the first jaw 431 can be opened or closed relative to the second jaw 432. The end effector 43 is of a single-opening type in which the first jaw 431 opens and closes relative to the second jaw 432.

The support shaft 431a includes a pair of shaft portions that are provided on both sides of the first jaw 431. The pair of shaft portions of the support shaft 431a are inserted in and rotatably supported by a pair of holes 432a provided to the second jaw 432. The connection portion 431b includes a long hole portion 431c. The long hole portion 431c comprises a pair of long holes 431c. A pin portion 47a is provided at the distal end of the rod 47 and extends in a direction orthogonal to the Y direction. One end and the other end of the pin portion 47a of the rod 47 are inserted in and supported by the pair of long holes 431c of the connection portion 431b of the first jaw 431 so that the pin portion 47a of the rod 47 is slidable along the long holes 431c of the connection portion 431b of the first jaw 431. When the rod 47 is moved in the Y1 direction, the pin portion 47a of the rod 47 slides along the long holes 431c, so that a force is applied in a direction in which the first jaw 431 opens. As a result, the first jaw 431 rotates in the opening direction about the axis of the support shaft 431a. To the contrary, when the rod 47 is moved in the Y2 direction, the pin portion 47a of the rod 47 slides along the long holes 431c, so that a force is applied in a direction in which the first jaw 431 closes. As a result, the first jaw 431 rotates in the closing direction about the axis of the support shaft 431a.

In an example illustrated in FIG. 12, the first jaw 431 is configured to move between the open position and the closed position relative to the second jaw 432 which is fixed.

Note that the second jaw 432 may be configured to move between the open position and the closed position relative to the first jaw 431 which is fixed, as illustrated in an example of FIG. 13.

As illustrated in FIGS. 11, 17 and 18, the rod 47 (elongate element) includes a first rod 47b connected to the first jaw 431, a flexible wire 47c connected to a proximal end of the first rod 47b, and a second rod 47d connected to a proximal end of the wire 47c. The wire 47c is provided inside the wrist joint 45. This allows the wire 47c to bend in response to articulation of the wrist joint 45, so that even when the wrist joint 45 articulates, the rod 47 (elongate element) can easily move the first jaw 431 and the second jaw 432 relative to each other between the open position and the closed position. Further, unlike a case where the rod 47 (elongate element) is entirely made of the wire 47c, the rod 47 includes the first rod 47b and the second rod 47d, so that the strength of the rod 47 (elongate element) can be maintained. The first rod 47b, the wire 47c, and the second rod 47d are electrically conductive. The wire 47c is an example of a "flexible wire."

The first rod 47b includes a pin portion 47a at a distal end of the first rod 47b, and moves the first jaw 431 by the force transmitted from the wire 47c and the second rod 47d. The wire 47c transmits the force transmitted from the second rod 47d to the first rod 47b. The second rod 47d transmits the force transmitted from the drive shaft 44d to the wire 47c.

The wire 47c is provided inside a flexible resin tube 47e. The resin tube 47e is a fluororesin-based tube. The resin tube 47e has a diameter substantially the same as that of the first rod 47b, eliminating a step at a connection position between the first rod 47b and the wire 47c. Further, an insulating coating 47f is provided on the outer surface of the second rod 47d.

Further, the wire 47c is a torque coil. This makes it possible to prevent the wire 47c from tightening or loosening depending on the rotation direction of the shaft 42 when the shaft 42 rotates. As a result, it is possible to prevent the movement distance of the rod 47 required for opening and closing the first jaw 431 and the second jaw 432 from differing depending on the degree of tightening of the wire 47c. Further, since the wire 47c is the torque coil, it is possible to achieve high torque transmission even in a bent state. The torque coil may be a single-directional twist that is made difficult to unravel by forming or the like, a combination of S-twist and Z-twist that provides equal torque transmission in both rotation directions, or the like.

As illustrated in FIG. 11, a flexible resin guide 90 is provided inside the wrist joint 45. The resin guide 90 is made of polyamide or silicone.

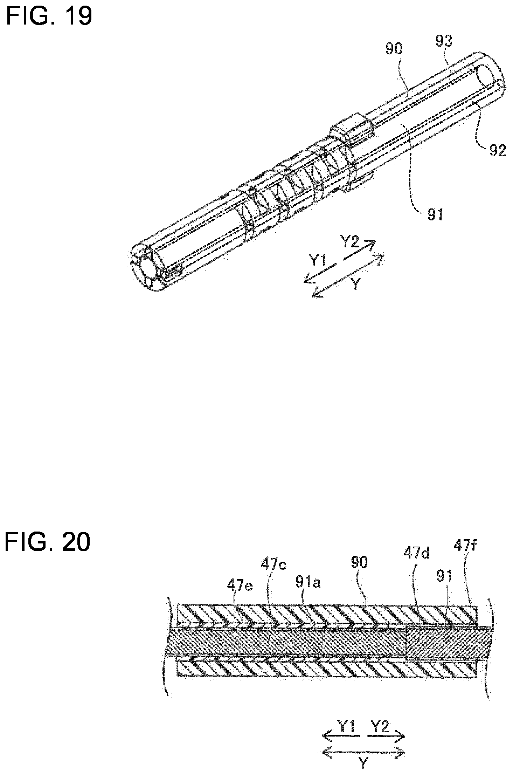

As illustrated in FIG. 19, the resin guide 90 includes a passage 91 passing through a longitudinal center line (a center line extending in the Y direction) of the resin guide 90 and guides the rod 47. This allows the rod 47 to be guided by the passage 91 of the resin guide 90, making it possible to easily move the rod 47 to open and close the first jaw 431 and the second jaw 432. Further, since the resin guide 90 is configured to be bent in accordance with the articulation of the wrist joint 45, even when the wrist joint 45 articulates, the rod 47 for opening and closing the first jaw 431 and the second jaw 432 can be easily moved. The resin guide 90 also includes passages 92 and 93 provided offset from the longitudinal center line of the resin guide 90. The passages 92 and 93 are provided for routing electrical leads.

As illustrated in FIG. 20, an inner surface 91a of the passage 91 of the resin guide 90, which guides the wire 47c, is made of fluororesin.

As illustrated in FIG. 11, the shaft 42 includes a positioning member 421 that positions the resin guide 90. As a result, the resin guide 90 can be positioned by the positioning member 421 of the shaft 42, so that the position of the resin guide 90 can be prevented from shifting due to the movement of the rod 47. The positioning member 421 includes a step portion 421a. The step portion 421a of the positioning member 421 and the first joint component 451 sandwich a protrusion 94 of the resin guide 90 therebetween, thereby positioning the resin guide 90 in the longitudinal direction of the resin guide 90. The protrusion 94 protrudes in a direction orthogonal to the longitudinal direction of the resin guide 90, and is sandwiched in the longitudinal direction of the resin guide 90 between the step portion 421a of the positioning member 421 and the first joint component 451.

As illustrated in FIGS. 7 to 9, the lever 48 that includes an arm 481 including a shaft portion 481a that is rotatably supported, and an engagement portion 482 that protrudes from the arm 481 and is engaged with the rod 47. The lever 48 is configured to rotate about an axis of the shaft portion 481a by the driving force from the fourth drive shaft 44d, so as to move the rod 47 in the Y direction. With this configuration, the shaft portion 481a of the lever 48, which moves the rod 47 in the Y direction, serves as a rotation axis of the lever 48, so there is no need to use one of the drive shafts 44a, 44b, 44c, and 44d as the rotation axis of the lever 48. Accordingly, the rod 47 can be moved in the Y direction of the shaft 42 using only one drive shaft (e.g., the fourth drive shaft 44d), to operate the end effector 43. As a result, the four drive shafts 44a to 44d can rotate the shaft 42 in roll, articulate the end effector 43 (the wrist joint 45) in pitch and yaw, and also move the rod 47 in the Y direction of the shaft 42 for operating the end effector 43.

The arm 481 includes a pair of arms 481 opposed to each other in the Z direction. The engagement portion 482 of one of the pair of arms 481 engages with the rod 47 from one side (the Z1 side). The engagement portion 482 of the other of the pair of arms 481 engages with the rod 47 from the other side (Z2 side). This allows the engagement portions 482 to engage with the rod 47 from the one side and the other side of the rod 47, so that the driving force of the fourth drive shaft 44d can be reliably transmitted to the rod 47 via the lever 48.

The pair of arms 481 are spaced apart from each other in the Z direction and connected to each other by a connection portion 483 extending in the Z direction. Each of the pair of arms 481 is provided to extend in the X direction from the connection portion 483 to the position of the rod 47. Each of the pair of arms 481 includes a shaft portion 481a between an end thereof on the connection portion 483 side and an end thereof on the rod 47 side. Specifically, each of the pair of arms 481 includes the shaft portion 481a at a position closer to the connection portion 483 from the center between the connection portion 483 side end portion and the rod 47 side end portion of the arm 481. Further, the rod 47 side end portion of each of the pair of arms 481 is provided with the engagement portion 482.

The engagement portion 482 of one of the pair of arms 481 protrudes in the Z2 direction and engages with an engagement portion 501 (described later) of the holding member 50 from the Z1 side. The engagement portion 482 of the other of the pair of arms 481 protrudes in the Z1 direction and engages with the engagement portion 501 of the holding member 59 from the Z2 side.

Further, the shaft portion 481a of one of the pair of arms 481 protrudes toward the Z1 side. The shaft portion 481a of the one of the pair of arms 481 is rotatably supported by a frame member 52 (see FIG. 7) provided inside the housing 41. Specifically, the shaft portion 481a of the one of the pair of arms 481 is inserted in and is rotatably supported by a support portion 521 including a hole provided in the frame member 52. The shaft portion 481a of the other of the pair of arms 481 protrudes toward the Z2 side. The shaft portion 481a of the other of the pair of arms 481 is rotatably supported by the base 411. Specifically, the shaft portion 481a of the other arm 481 is inserted in and rotatably supported by a support portion 411a including a recess provided in the base 411.

Further, the axis of the shaft portion 481a is located between the engagement portion 482 and the fourth drive shaft 44d in the X direction orthogonal to the Y direction. This allows the axis of the shaft portions 481a to be positioned between the engagement portion 482 and the fourth drive shaft 44d in the X direction orthogonal to the Y direction, making the lever 48 more compact than in a case where the axis of the shaft portions 481a is positioned on the outer side of the fourth drive shaft 44d. The axis of the shaft portions 481a is disposed on the fourth drive shaft 44d side in the X direction from the center between the engagement portion 482 and the fourth drive shaft 44d.

Further, the driving force transmission part 49 is provided between the fourth driving shaft 44d and the lever 48, and transmits the driving force from the fourth driving shaft 44d to the lever 48. This allows the driving force to be transmitted from the fourth drive shaft 44d to the lever 48 via the driving force transmission part 49, so that the lever 48 can be arranged away from the fourth drive shaft 44d. As a result, even in cases where it is difficult to secure a space for providing the lever 48 near the fourth drive shaft 44d, the space for providing the lever 48 can be easily secured.

The driving force transmission part 49 includes a gear train 491 including a spur gear 491a and a sector gear 491b, and transmits the driving force from the fourth drive shaft 44d to the lever 48 by the gear train 491. This allows the driving force to be reliably transmitted from the fourth drive shaft 44d to the lever 48 by the gear train 491, which transmits the driving force through meshing. Further, since the gear train 491 includes the sector gear 491b, the gear train 491 can be made more compact than in a case where a spur gear is provided instead of the sector gear 491b.

Further, the driving force transmission part 49 transmits the rotation of the fourth drive shaft 44d to the lever 48 with decelerating the speed of the rotation of the fourth drive shaft 44d. As a result, the lever 48 is driven with a torque that increases inversely proportional to the deceleration of the rotation of the fourth drive shaft 44d, so that the rod 47 can be easily moved in the Y direction by driving the lever 48. In addition, the driving of the lever 48 by the torque that increases inversely proportional to the deceleration of the rotation of the fourth drive shaft 44d is effective in the case where the spring member 51 is provided as in an embodiment.

The sector gear 491b meshes with a spur gear 44d1 provided to the fourth drive shaft 44d. The sector gear 491b has the number of teeth that reduces the rotation speed of the fourth drive shaft 44d. The spur gear 491a meshes with a sector gear 483a provided to the connection portion 483 of the lever 48. The spur gear 491a and the sector gear 491b are supported by a common rotation shaft 491c and rotate integrally with each other by the rotation shaft 491c.

In addition, the holding member 50 holds the rod 47. The spring member 51 biases the holding member 50 toward the distal end side (Y1 side) in the Y direction. The engagement portions 482 of the arms 481 of the lever 48 are engaged with the rod 47 via the holding member 50, and a gripping force of the end effector 43 is generated by the reaction force of the spring member 51 when the engagement portions 482 push the holding member 50 toward the proximal end side (Y2 side) in the Y direction. Accordingly, it is possible to determine the gripping force of the end effector 43 by a spring constant of the spring member 51, so that the gripping force of the end effector 43 can be applied stably. Also, unlike a case where the gripping force of the end effector 43 is applied using a wire(s) as in the wrist joint 45, the gripping force is less susceptible to slight stretching of the wire.

The holding member 50 includes the engagement portion 501, a spring accommodation portion 502, and a rod holding portion 503. The engagement portion 501 of the holding member 50 is engaged with the engagement portions 482 of the lever 48. The engagement portion 501 is configured as a recess formed in a circumferential shape. A Z1 side portion of the engagement portion 501 is recessed toward the Z2 side, and is engaged with the engagement portion 482 of one of the pair of arms 481. A Z2 side portion of the engagement portion 501 is recessed toward the Z1 side, and is engaged with the engagement portion 482 of the other of the pair of arms 481. The spring accommodation portion 502 accommodates the spring member 51. The spring accommodation portion 502 includes a recess recessed toward the Y1 side. The spring accommodation portion 502 holds the spring member 51 between the spring accommodation portion 502 and a plate-shaped washer 53 provided on the Y2 side of the spring accommodation portion 502. The rod holding portion 503 holds a held portion 47g provided to the rod 47. The rod holding portion 503 includes a hole extending along the Y direction.

The spring member 51 is a compression coil spring having a predetermined spring constant. When the holding member 50 is moved in the Y2 direction by the lever 48, the spring member 51 is compressed between the spring accommodation portion 502 and the washer 53. When the holding member 50 is moved in the Y1 direction by the lever 48, the spring member 51 is stretched between the spring accommodation portion 502 and the washer 53.

Next, an operation of moving the rod 47 in the Y direction by the lever 48 is described. When the fourth drive shaft 44d is driven to rotate, the driving force from the fourth drive shaft 44d is transmitted to the lever 48 via the gear train 491 of the driving force transmission part 49. This causes the lever 48 to rotate about the axis of the shaft portions 481a. When the rod 47 is moved in the Y1 direction, a Y1 side surface of the engagement portion 482 of the lever 48 abuts against a Y2 side surface of the engagement portion 501 of the holding member 50, and the holding member 50 is pushed and moved in the Y1 direction. With this, the rod 47, whose held portion 47g is held by the rod holding portion 503 of the holding member 50, is moved in the Y1 direction. In this case, the first jaw 431 is moved in the opening direction. To the contrary, when the rod 47 is moved in the Y2 direction, a Y2 side surface of the engagement portion 482 of the lever 48 abuts against a Y1 side surface of the engagement portion 501 of the holding member 50, and the holding member 50 is pushed and moved in the Y2 direction. With this, the rod 47, whose held portion 47g is held by the rod holding portion 503 of the holding member 50, is moved in the Y2 direction. In this case, the first jaw 431 is moved in the closing direction.

### (Configuration of Mechanism for Fixing Memory Board)

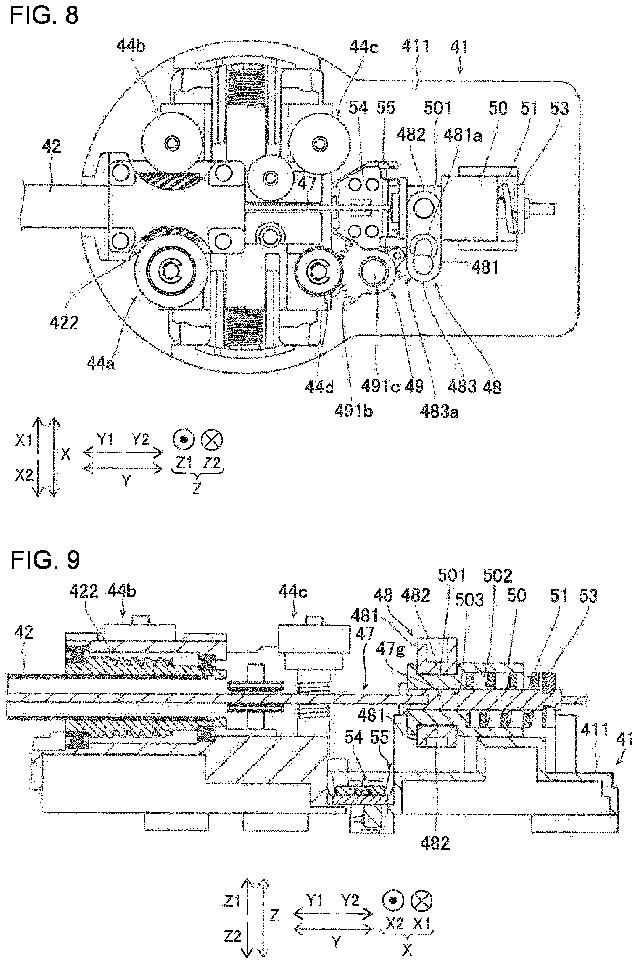

Next, a configuration of a mechanism 55 for fixing a circuit board 54 is described with reference to FIGS. 8, 21, and 22.

As illustrated in FIGS. 8, 21, and 22, the surgical instrument 40a includes the circuit board 54 and the fixing mechanism 55 for fixing the circuit board 54. The circuit board 54 is provided on the base 411. The circuit board 54 is a memory board. The circuit board 54 stores information about the surgical instrument 40a, such as a type of the surgical instrument 40a and a number of times the surgical instrument 40a has been used. The fixing mechanism 55 fixes the circuit board 54 to the base 411. The fixing mechanism 55 is a snap-fit fixing mechanism with a plurality (four pieces) of snap fit portions 551. The snap fit portions 551 are provided two on each of the Y1 side and the Y2 side of the circuit board 54, and press an outer periphery of the circuit board 54 from the Z1 side. Further, when the circuit board 54 is attached to the fixing mechanism 55 from the Z1 side toward the Z2 side, the snap fit portions 551 are pressed by the circuit board 54 and elastically deformed. By providing the snap-fit fixing mechanism as the fixing mechanism 55, it is possible to fix the circuit board 54 to the base 411 with a simple structure.

### (Configuration of Wrist Joint)

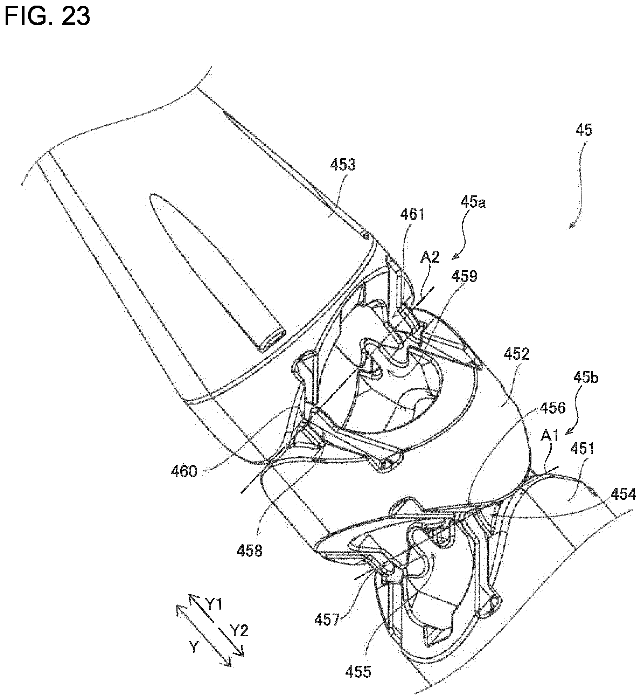

Next, a configuration of the wrist joint 45 is described with reference to FIGS. 23 to 32.

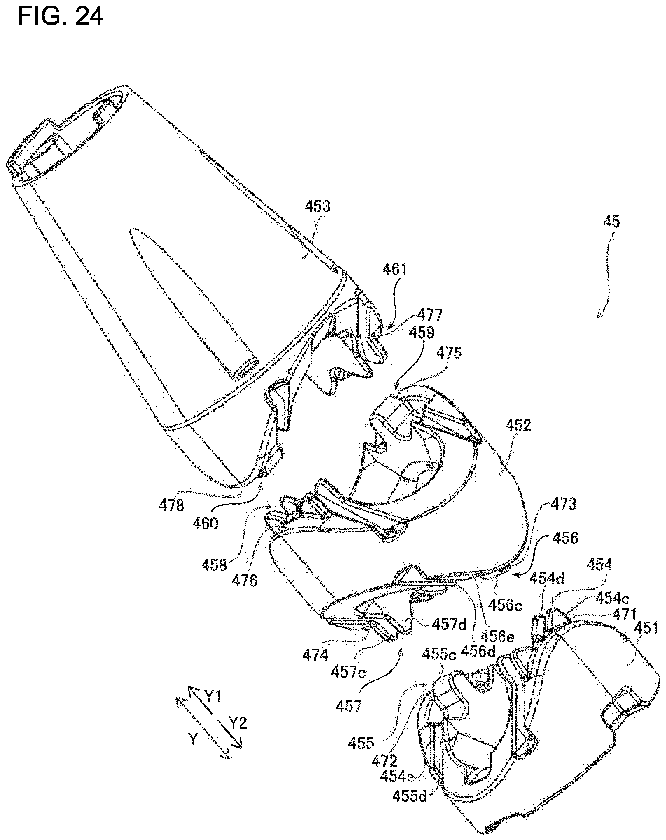

As illustrated in FIGS. 23 and 24, the wrist joint 45 includes the first joint component 451 and the second joint component 452 that is adjacent to the first joint component 451 on the distal side (Y1 side) and rotates relative to the first joint component 451. The wrist joint 45 also includes the third joint component 453 that is adjacent to the second joint component 452 on the distal side (Y1 side) and rotates relative to the second joint component 452. As illustrated in FIGS. 10 and 23, the second joint component 452 is rotatable relative to the first joint component 451 about the rotation axis A1. The rotation axis A1 extends in a direction orthogonal to the longitudinal direction (Y direction) of the shaft 42. Further, the third joint component 453 is rotatable relative to the second joint component 452 about the rotation axis A2 that is orthogonal to the rotation axis A1. The rotation axis A2 extends in a direction orthogonal to the rotation axis A1 when viewed in the longitudinal direction (Y direction) of the shaft 42. As illustrated in FIG. 24, the wrist joint 45 includes the first joint 45a that rotates about the rotation axis A1, and the second joint 45b that rotates about the rotation axis A2.

The first joint component 451 includes a first meshing portion 454, a second meshing portion 455 arranged so as to be opposed to the first meshing portion 454 in a radial direction (A1 direction) orthogonal to the longitudinal direction of the shaft, a first contact portion 471 located radially outside of and adjacent to the first meshing portion 454, and a second contact portion 472 provided radially outside of and adjacent to the second meshing portion 455. The first meshing portion 454, the second meshing portion 455, the first contact portion 471, and the second contact portion 472 are provided at the end portion on the distal side (the Y1 side) of the first joint component 451.

The second joint component 452 includes a third meshing portion 456 that meshes with the first meshing portion 454, a fourth meshing portion 457 that is provided so as to be opposed to the third meshing portion 456 in the radial direction (A1 direction) and meshes with the second meshing portion 455, a third contact portion 473 that is in contact with the first contact portion 471, and a fourth contact portion 474 that is in contact with the second contact portion 472. The third meshing portion 456, the fourth meshing portion 457, the third contact portion 473 and the fourth contact portion 474 are provided at the end portion on the proximal side (Y2 side) of the second joint component 452. The contact between the first contact portion 471 and the third contact portion 473 reduces the load between the first meshing portion 454 and the third meshing portion 456. The contact between the second contact portion 472 and the fourth contact portion 474 reduces the load between the second meshing portion 455 and the fourth meshing portion 457.

The second joint component 452 also includes a fifth meshing portion 458, a sixth meshing portion 459 provided so as to be opposed to the fifth meshing portion 458 in the radial direction (A2 direction), a fifth contact portion 475 located radially outward of and adjacent to the fifth meshing portion 458, and a sixth contact portion 476 provided radially outside of and adjacent to the sixth meshing portion 459. The fifth meshing portion 458, the sixth meshing portion 459, the fifth contact portion 475 and the sixth contact portion 476 are provided at the end portion on the distal side (the Y1 side) of the second joint component 452.

The third joint component 453 includes a seventh meshing portion 460 that meshes with the fifth meshing portion 458, an eighth meshing portion 461 that is arranged so as to be opposed to the seventh meshing portion 460 in the radial direction (A2 direction) and meshes with the sixth meshing portion 459, a seventh contact portion 477 that is in contact with the fifth contact portion 475, and an eighth contact portion 478 that is in contact with the sixth contact portion 476. The seventh meshing portion 460, the eighth meshing portion 461, the seventh contact portion 477, and the eighth contact portion 478 are provided at the end portion on the proximal side (the Y2 side) of the third joint component 453. The contact between the fifth contact portion 475 and the seventh contact portion 477 reduces the load between the fifth meshing portion 458 and the seventh meshing portion 460. The contact between the sixth contact portion 476 and the eighth contact portion 478 reduces the load between the sixth meshing portion 459 and the eighth meshing portion 461.

In an embodiment, as illustrated in FIG. 24, the first meshing portion 454 and the second meshing portion 455 have different shapes from each other. The third meshing portion 456 and the fourth meshing portion 457 have different shapes from each other. This makes it difficult for the first meshing portion 454 of the first joint component 451 and the fourth meshing portion 457 of the second joint component 452 to mesh with each other, and makes it difficult for the second meshing portion 455 of the first joint component 451 and the third meshing portion 456 of the second joint component 452 to mesh with each other. This allows the first and third meshing portions 454 and 456 to be properly meshed, and the second and fourth meshing portions 455 and 457 to be properly meshed, when the first and second joint components 451 and 452 are assembled together. As a result, the wrist joint can be easily and accurately assembled, and even if a problem occurs, the cause of the problem can be easily identified, allowing for appropriate quality control of the surgical instrument.

Further, the fifth meshing portion 458 and the sixth meshing portion 459 have shapes different from each other. The seventh meshing portion 460 and the eighth meshing portion 461 have shapes different from each other. This also enables accurate assembly of the wrist joint and appropriate quality control of the surgical instrument.

Further, in an embodiment, the first meshing portion 454 and the fourth meshing portion 457 have the same shape as each other. The second meshing portion 455 and the third meshing portion 456 have the same shape as each other. This allows the meshing structure of the first meshing portion 454 and the third meshing portion 456 to be the same as the meshing structure of the second meshing portion 455 and the fourth meshing portion 457, so that the joint movement of the wrist joint 45 can be supported in a well-balanced manner by the two meshing structures between the first joint component 451 and the second joint component 452.

The fifth meshing portion 458 and the eighth meshing portion 461 have the same shape as each other. The sixth meshing portion 459 and the seventh meshing portion 460 have the same shape as each other. This also makes it possible to support the joint movement of the wrist joint 45 in a well-balanced manner with the two meshing structures between the second joint component 452 and the third joint component 453.

Moreover, the first meshing portion 454, the fourth meshing portion 457, the fifth meshing portion 458, and the eighth meshing portion 461 have the same shape as one another. The second meshing portion 455, the third meshing portion 456, the sixth meshing portion 459 and the seventh meshing portion 460 have the same shape as one another. In other words, the first meshing portion 454 and the fifth meshing portion 458 have the same configuration as each other, and the second meshing portion 455 and the sixth meshing portion 459 have the same configuration as each other. Further, the third meshing portion 456 and the seventh meshing portion 460 have the same configuration as each other, and the fourth meshing portion 457 and the eighth meshing portion 461 have the same configuration as each other. Accordingly, the description of the fifth meshing portion 458, the sixth meshing portion 459, the seventh meshing portion 460 and the eighth meshing portion 461 will be omitted as appropriate for eliminating redundancy.

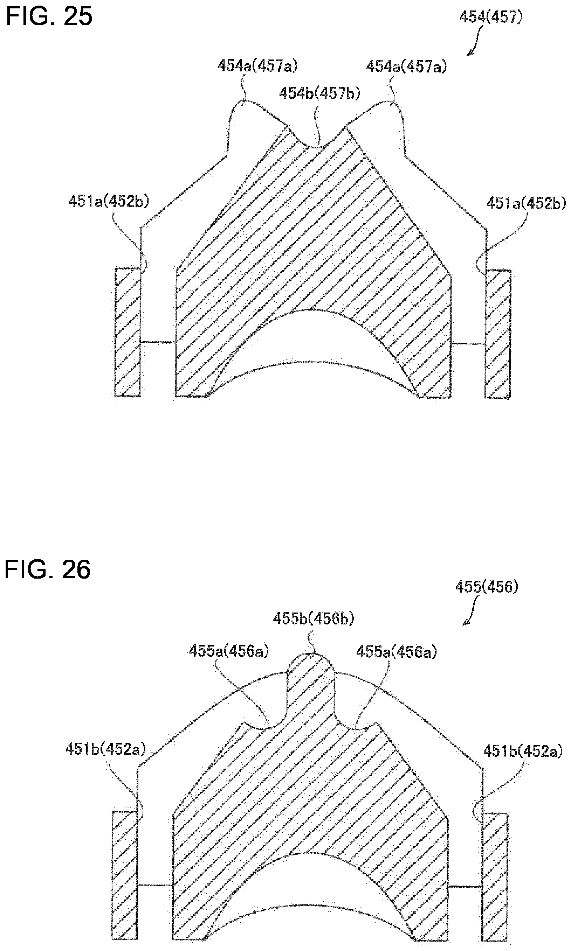

As illustrated in FIG. 25, the first meshing portion 454 includes a pair of first protrusions 454a arranged along the rotation direction of the second joint component 452, and a first recess 454b arranged between the pair of first protrusions 454a. In the same manner, the fourth meshing portion 457 includes a pair of fourth protrusions 457a arranged along the rotation direction of the second joint component 452, and a fourth recess 457b arranged between the pair of fourth protrusions 457a.

As illustrated in FIG. 26, the second meshing portion 455 includes a pair of second recesses 455a arranged along the rotation direction of the second joint component 452, and a second protrusion 455b arranged between the pair of second recesses 455a. In the same manner, the third meshing portion 456 includes a pair of third recesses 456a arranged along the rotation direction of the second joint component 452, and a third protrusion 456b arranged between the pair of third recesses 456a.

This makes it possible to prevent the first meshing portion 454 having the first recess 454b and the fourth meshing portion 457 having the fourth recess 457b from easily meshing with each other, and also makes it possible to prevent the second meshing portion 455 having the second protrusion 455b and the third meshing portion 456 having the third protrusion 456b from easily meshing with each other. As a result, it is possible to effectively prevent the first joint component 451 and the second joint component 452 from being assembled in a wrong direction.

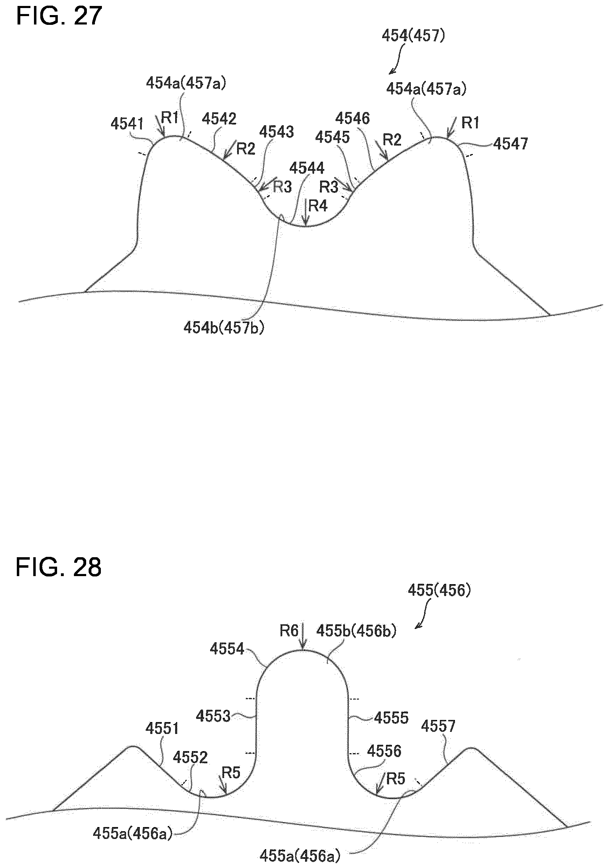

As illustrated in FIGS. 27 and 28, each of the first meshing portion 454, the second meshing portion 455, the third meshing portion 456 and the fourth meshing portion 457 have a shape in which arcs are connected together, or a shape in which arcs and straight lines are connected together, when viewed in the rotation axis direction of the second joint component 452 (A1 direction). This makes it possible to easily design the first meshing portion 454, the second meshing portion 455, the third meshing portion 456, and the fourth meshing portion 457. In addition, the shapes of the first meshing portion 454, the second meshing portion 455, the third meshing portion 456, and the fourth meshing portion 457 can be easily inspected, so that the shapes of the first meshing portion 454, the second meshing portion 455, the third meshing portion 456, and the fourth meshing portion 457 can be more appropriately managed.

Specifically, the first meshing portion 454 and the fourth meshing portion 457 each have a shape in which arcs are connected, as illustrated in FIG. 27. The first meshing portion 454 and the fourth meshing portion 457 each have the shape (the outer shape) formed by connecting lines including an arc 4541 having a radius R1, an arc 4542 having a radius R2 connected to the arc 4541, an arc 4543 having a radius R3 connected to the arc 4542, an arc 4544 having a radius R4 connected to the arc 4543, an arc 4545 having a radius R3 connected to the arc 4544, an arc 4546 having a radius R2 connected to the arc 4545, and an arc 4547 having a radius R1 connected to the arc 4546. Further, the first meshing portion 454 and the fourth meshing portion 457 each have a linear symmetric shape when viewed along the rotation axis direction of the second joint component 452 (A1 direction).

As illustrated in FIG. 28, the second and third meshing portions 455 and 456 each have a shape in which arcs and straight lines are connected. The second meshing portion 455 and the third meshing portion 456 each have an outer shape formed by connecting lines including a line segment 4551, an arc 4552 having a radius R5 connected to line segment 4551, a line segment 4553 connected to the arc 4552, an arc 4554 having a radius R6 connected to the line segment 4553, a line segment 4555 connected to the arc 4554, an arc 4556 having the radius R5 connected to the line segment 4555, and a line segment 4557 connected to the arc 4556. Further, the second meshing portion 455 and the third meshing portion 456 each have a linear symmetric shape when viewed in the rotation axis direction (A1 direction) of the second joint component 452.

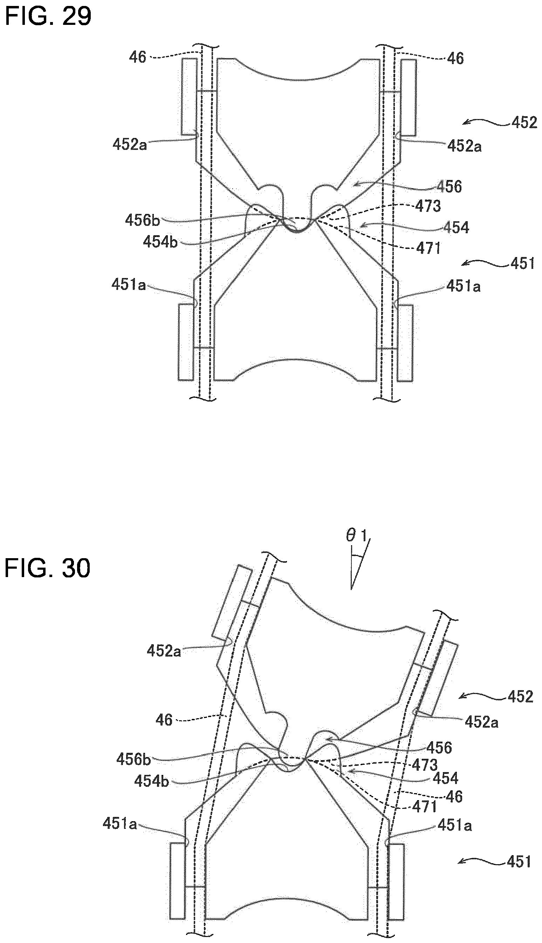

As illustrated in FIGS. 29 to 32, the wrist joint 45 is articulated by driving the wires 46 to rotate the second joint component 452 relative to the first joint component 451. As illustrated in FIG. 10, the wire 46 has the end portion 46e thereof fixed to the third joint component 453 on the end effector side (Y1 side) of the wrist joint 45.

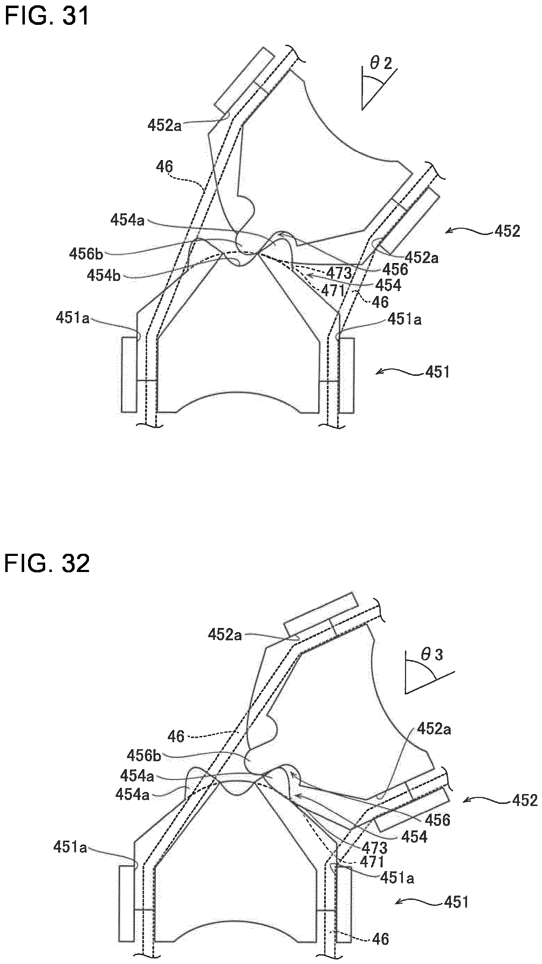

As illustrated in FIG. 24, the first meshing portion 454 includes a first portion 454c and a second portion 454d that are separated in the radial direction (A1 direction) by a distance that allows the wires 46 to pass therethrough. Further, each of the first portion 454c and the second portion 454d is formed with the first protrusions 454a and the first recess 454b. In the same manner as the first meshing portion 454, the fourth meshing portion 457 includes a third portion 457c and a fourth portion 457d that are separated in the radial direction (A1 direction) by a distance that allows the wires 46 to pass through. Further, each of the third portion 457c and the fourth portion 457d is formed with the fourth protrusions 457a and the fourth recess 457b. This prevents the wires 46 from interfering with the first portion 454c and the second portion 454d when the joint movement performed in which the second joint component 452 is rotated significantly relative to the first joint component 451 such as being illustrated in FIG. 32.

As illustrated in FIG. 24 the second meshing portion 455 includes a fifth portion 455c that is continuously formed in the radial direction (A1 direction). The second meshing portion 455 also includes a portion 455d and a portion 455e that are separated in the radial direction (A1 direction) by a distance that allows the wires 46 to pass therethrough. Further, the second protrusion 455b is formed in the fifth portion 455c. Further, the second recesses 455a are formed in each of the portion 455d and the portion 454e.

As in the second meshing portion 455, the third meshing portion 456 includes a sixth portion 456c that is formed continuously in the radial direction (A1 direction). The third meshing portion 456 also includes a portion 456d and a portion 456e that are separated in the radial direction (A1 direction) by a distance that allows the wires 46 to pass therethrough. Further, the third protrusion 456b is formed in the sixth portion 456c. Further, the third recesses 456a are formed in each of the portion 456d and the portion 456e.

As illustrated in FIG. 25, the first joint component 451 includes first through holes 451a which are disposed at positions spaced apart from the first meshing portion 454 and through which the wires 46 pass. As illustrated in FIG. 26, the first joint component 451 includes second through holes 451b which are disposed at positions spaced apart from the second meshing portion 455 and through which the wires 46 passes. As illustrated in FIG. 26, the second joint component 452 includes third through holes 452a which are disposed at positions spaced apart from the third meshing portion 456 and through which the wires 46 pass. As illustrated in FIG. 25, the second joint component 452 includes fourth through holes 452b which are disposed at positions spaced apart from the fourth meshing portion 457 and through which the wires 46 pass. This allows the through holes through which the wires 46 pass to be located at the positions away from the meshing portions, thereby preventing the meshing portions from interfering with the wires 46.

Next, with reference to FIGS. 29 to 32, the rotation of the second joint component 452 relative to the first joint component 451 is described. Although FIGS. 29 to 32 illustrate the relationship between the first meshing portion 454 of the first joint component 451 and the third meshing portion 456 of the second joint component 452, the relationship between the second meshing portion 455 of the first joint component 451 and the fourth meshing portion 457 of the second joint component 452 is in a same manner with the protrusions and the recesses interchanged, and thus illustration and description thereof is omitted for eliminating redundancy. Also the rotation of the third joint component 453 relative to the second joint component 452 takes the same or similar operation, and thus illustration and description thereof is omitted for eliminate redundancy.

As illustrated in FIG. 29, when the second joint component 452 is not rotated relative to the first joint component 451, the third protrusion 456b of the third meshing portion 456 of the second joint component 452 is fit in the first recess 454b of the first meshing portion 454 of the first joint component 451.

As illustrated in FIG. 30, by the operation of the wires 46, the third protrusion 456b of the third meshing portion 456 of the second joint component 452 is rotated with respect to the first recess 454b of the first meshing portion 454 of the first joint component 451 while contacting with the first recess 454b, so that the second joint component 452 is rotated by an angle θ1 relative to the first joint component 451.

As illustrated in FIG. 31, by the operation of the wires 46, the third protrusion 456b of the third meshing portion 456 of the second joint component 452 is rotated with respect to the first protrusion 454a of the first meshing portion 454 of the first joint component 451 while contacting with the first protrusion 454a, so that the second joint component 452 is rotated by an angle θ2 relative to the first joint component 451. The angle θ2 is greater than the angle θ1.

As illustrated in FIG. 32, by the operation of the wires 46, the third protrusion 456b of the third meshing portion 456 of the second joint component 452 is further rotated with respect to the first protrusion 454a of the first meshing portion 454 of the first joint component 451 while contacting with the first protrusion 454a, so that the second joint component 452 is rotated by an angle θ3 relative to the first joint component 451. However, the angle θ3 is greater than the angle θ2.

### (Modifications)

Note that one or more embodiments disclosed herein should be considered as exemplary in all respects and do not limit the invention. The scope of the invention is indicated by claims, not by explanation of one or more embodiments described above, and includes equivalents to the claims and all alterations (modification) within the same.

For example, in one or more embodiments described above, a case has been described in which a single-opening configuration in which the first jaw member of the end effector opens and closes relative to the second jaw member, but the invention is not limited to this. In the invention, the end effector may have a double-opening configuration in which the first jaw member and the second jaw member each open and close. Also, the end effector may have a mechanism that moves a second jaw member to open and close relative to a fixed first jaw member. In this case, the distal end of the rod of the elongate element may be connected to the proximal end of the second jaw member, and the second jaw member may be moved to open and close by the movement of the elongate element in the longitudinal direction of the shaft.

Further, in one or more embodiments described above, a case has been described in which the diameter D1 is larger than 1/2 and smaller than 3/4 of the diameters D2 and D3, but the invention is not limited to this. In the invention, the diameter D1 may be equal to or less than 1/2 of the diameters D2 and D3, or may be equal to or more than 3/4 of the diameters D2 and D3.

Further, in one or more embodiments described above, a case has been described in which the diameter D1 is 4 mm or more and 6 mm or less, and the diameters D2 and D3 are 7 mm or more and 9 mm or less, but the invention is not limited to this. In the invention, the diameter D1 may be smaller than 4 mm or larger than 6 mm. Further, the diameters D2 and D3 may be smaller than 7 mm or larger than 9 mm.

Further, in one or more embodiments described above, a case has been described in which the diameter D4 is substantially the same as the diameter D1, but the invention is not limited to this. In the invention, the diameter D4 may be different from the diameter D1.

Further, in one or more embodiments described above, a case has been described in which the heat shrink tube covers 90% to 100% of the metal shaft in the longitudinal direction of the metal shaft, but the invention is not limited to this. In the invention, the heat shrink tube may cover less than 90% of the metal shaft in the longitudinal direction of the metal shaft.

Further, in one or more embodiments described above, a case has been described in which the metal shaft is the stainless steel pipe, but the invention is not limited to this. In the invention, the metal shaft may be a metal pipe other than stainless steel.

Further, in one or more embodiments described above, a case has been described in which the heat shrink tube is the polyethylene-based heat shrink tube or the fluororesin-based heat shrink tube, but the invention is not limited to this. In the invention, the heat shrink tube may be a tube other than the polyethylene-based heat shrink tube or the fluororesin-based heat shrink tube.

Further, in one or more embodiments described above, a case has been described in which the heat shrink tube is the single-layered heat shrink tube, but the invention is not limited to this. In the invention, the heat shrink tube may be a double-layered heat shrink tube.

Further, in one or more embodiments described above, a case has been described in which the heat shrink tube is colored. However, the invention is not limited to this. In the invention, the heat shrink tube may be colorless.

Further, in one or more embodiments described above, a case has been described in which the wrist joint (the wrist joint portion) is configured to be articulated in the plurality of degrees of freedom, but the invention is not limited to this. In the invention, the wrist joint portion may be configured to articulate in one degree of freedom.

Further, in one or more embodiments described above, a case has been described in which the first meshing portion and the second meshing portion have different shapes from each other, and the third meshing portion and the fourth meshing portion have different shapes from each other, but the invention is not limited to this. In the invention, the first and second meshing portions may have the same shape as each other, and the third and fourth meshing portions may have the same shape as each other.

### (Aspects)

It will be understood by those skilled in the art that the exemplary embodiments described above are embodiments of the following aspects.

(Item 1) A surgical instrument for a robotic surgical system comprising:
an end effector;
a wrist joint portion connected to the end effector;
a shaft connected to the wrist joint portion; and
an interface that supports a proximal end side of the shaft and configured to be attached to a robot arm of the robotic surgical system, wherein
the shaft includes a hollow metal shaft and a heat shrink tube covering a surface of the metal shaft.

(Item 2) The surgical instrument according to item 1, wherein
the heat shrink tube covers 90% or more and 100% or less of the metal shaft in a longitudinal direction of the metal shaft.

(Item 3) The surgical instrument according to item 1 or 2, wherein
the heat shrink tube has an electrical insulation.

(Item 4) The surgical instrument according to any one of items 1 to 3, wherein
the metal shaft is a stainless steel pipe; and
the heat shrink tube is a polyethylene-based heat shrink tube or a fluororesin-based heat shrink tube.

(Item 5) The surgical instrument according to any one of items 1 to 4, wherein
the heat shrink tube is a single layer heat shrink tube.

(Item 6) The surgical instrument according to any one of items 1 to 5, wherein
the heat shrink tube has a color different from the color of the metal shaft.

(Item 7) The surgical instrument according to any one of items 1 to 6, wherein
the wrist joint portion is configured to articulate with a plurality of degrees of freedom.

(Item 8) The surgical instrument according to any one of items 1 to 7, wherein
the wrist joint portion includes a first joint rotating about a first rotation axis intersecting the longitudinal direction of the shaft, and a second joint rotating about a second rotation axis intersecting both the longitudinal direction and the first rotation axis.

(Item 9) The surgical instrument according to any one of items 1 to 8, further comprising an elongate element; wherein
the end effector includes first and second jaw members that move between an open position and a closed position; and
the elongate element is configured to move in the longitudinal direction of the shaft to move at least one of the first and second jaw members.

(Item 10) The surgical instrument according to item 9, wherein
the elongate member includes a first rod connected to at least one of the first and second jaw members, a wire connected to a proximal end of the first rod, and a second rod connected to a proximal end of the wire, and
the wire has flexibility and is arranged inside the wrist joint portion.

(Item 11) The surgical instrument according to item 10, wherein
the conductive wire is a conductive torque coil.

(Item 12) The surgical instrument according to any one of items 9 to 11, further comprising a flexible resin guide is arranged inside the wrist joint portion, wherein
the resin guide includes a passage formed to pass through a longitudinal centerline of the resin guide, and
the wire passes through the passage.

(Item 13) The surgical instrument according to item 12, wherein
the shaft includes a positioning member that positions the resin guide.

(Item 14) The surgical instrument according to any one of items 1 to 13, further comprising:
an interface that is provided to a proximal end side of the shaft and configured to be attached to an instrument attachment portion of a robot arm of the robotic surgical system, wherein
the instrument attachment portion includes a first drive member and a second drive member, and
the interface includes a first reception member that receives a driving force from the first drive member to drive the end effector, and a second reception member that receives a driving force from the second drive member to drive the wrist joint portion.

## Claims

1. A surgical instrument (40a) for a robotic surgical system (100) comprising:
an end effector (43) including first and second jaw members (431, 432) movable between an open position and a closed position, wherein at least one of the first and second jaw members is rotatably provided about a rotation axis (431a);
a wrist joint portion (45) connected to a proximal end of the end effector; and
a shaft (42) connected to a proximal end of the wrist joint portion, wherein
a diameter D1 of the end effector at a portion where the rotation axis is provided is smaller than a diameter D2 of a central portion in a longitudinal direction of the wrist joint portion and a diameter D3 of a central portion in the longitudinal direction of the shaft.

2. The surgical instrument according to claim 1, wherein
the diameter D1 is greater than 1/2 of the diameters D2 and D3 and smaller than 3/4 of the diameters D2 and D3.

3. The surgical instrument according to claim 1 or 2, wherein
the diameter D1 is equal to or greater than 4 mm and equal to or less than 6 mm, and the diameter D2 and the diameter D3 are equal to or greater than 7 mm and equal to or less than 9 mm.

4. The surgical instrument according to any one of claims 1 to 3, wherein
the wrist joint portion includes a connection portion (45c) to which the end effector is connected, and a diameter D4 of a distal end of the connection portion is smaller than the diameters D2 and D3.

5. The surgical instrument according to claim 4, wherein
the connection portion of the wrist joint portion has the diameter D4 at the distal end of the connection portion and the diameter D2 at a proximal end of the connection portion.

6. The surgical instrument according to claim 4 or 5, wherein
the connection portion of the wrist joint portion has a shape that gradually tapers from the proximal end having the diameter D2 to the distal end having the diameter D4.

7. The surgical instrument according to any one of claims 4 to 6, wherein
the diameter D4 is substantially the same as the diameter D1.

8. The surgical instrument according to any one of claims 4 to 7, wherein
the wrist joint portion includes a first joint (45a) between a first member (453) including the connection portion and a second member (452), and includes a second joint (45b) between the second member and a third member (451),
the first joint is configured to articulate about a first rotation axis (A2) that intersects the longitudinal direction of the shaft, and
the second joint is configured to articulate about a second rotation (A1) axis that intersects both the longitudinal direction of the shaft and the first rotation axis.

9. The surgical instrument according to any one of claims 1 to 8, wherein
an elongate element (47) configured to move in the longitudinal direction of the shaft to cause the relative movement of the first and second jaw members,
the elongate element comprises a first rod (47b) connected to at least one of the first and second jaw members, a flexible wire (47c) connected to a proximal end of the first rod, and a second rod (47d) connected to a proximal end of the flexible wire, and
the flexible wire is provided inside the wrist joint portion.

10. The surgical instrument according to claim 9, wherein
the flexible wire is a torque coil.

11. The surgical instrument according to claim 10, wherein
a flexible resin guide (90) is provided inside the wrist joint portion,
the resin guide includes a passage (91) that passes through a longitudinal center line of the resin guide and guides the elongate element, and
the shaft includes a positioning member (421) that positions the resin guide.

12. The surgical instrument according to any one of claims 1 to 11, wherein
the shaft comprises a hollow metal shaft (42a) and an insulating heat shrink tube (42b) covering a surface of the metal shaft,
the heat shrink tube covers 90% or more and 100% or less of the metal shaft in a longitudinal direction of the metal shaft.

13. The surgical instrument according to claim 12, wherein
the heat shrink tube is a single layer heat shrink tube.

14. The surgical instrument according to claim 12 or 13, wherein
the metal shaft is a stainless steel pipe, and
the heat shrink tube is a polyethylene-based heat shrink tube or a fluororesin-based heat shrink tube.

15. The surgical instrument according to any one of claims 1 to 14, further comprising:
an interface (41) that is provided to a proximal end side of the shaft and configured to be attached to an instrument attachment portion (212) of a robot arm (21a) of the robotic surgical system, wherein
the instrument attachment portion includes a first drive member (214d) and a second drive member (214b, 214c), and
the interface includes a first reception member (442) that receives a driving force from the first drive member to drive the end effector, and a second reception member (442) that receives a driving force from the second drive member to drive the wrist joint portion.
